# EUROPEAN PATENT SPECIFICATION

(11) **EP 2 393 418 B1**
(45) Date of publication and mention of the grant of the patent: **21.03.2018**
(21) Application number: 10739031.2
(22) Date of filing: 02.02.2010
(51) Int. Cl.: A61B 5/00, A61B 5/145, A61B 5/1486, A61M 5/158, A61M 5/32

(54) **COMPACT ON-BODY PHYSIOLOGICAL MONITORING DEVICES**
AM KÖRPER ZU TRAGENDE KOMPAKTE VORRICHTUNGEN ZUR ÜBERWACHUNG PHYSIOLOGISCHER PARAMETER
DISPOSITIFS DE SURVEILLANCE PHYSIOLOGIQUE DE FAIBLE ENCOMBREMENT DISPOSÉS SUR L'ORGANISME

(30) Priority: 03.02.2009 US 149639 P; 01.02.2010 US 698124; 01.02.2010 US 698129
(43) Date of publication of application: 14.12.2011
(60) Divisional application: 17201183.5
(73) Proprietor: Abbott Diabetes Care Inc., Alameda, CA 94502 (US)
(72) Inventor: THOMAS, Christopher, Allen, San Leandro CA 94579 (US); HOSS, Udo, Castro Valley CA 94522 (US); FENNELL, Martin, J., Concord CA 94521 (US); HE, Lei, Moraga, CA 94556 (US); LOVE, Michael, Pleasanton CA 94566 (US); YEE, Phillip, San Francisco CA 94122 (US)
(74) Representative: Kazi, Ilya
(86) International application number: PCT/US2010/022928
(87) International publication number: WO 2010/091028

(56) References cited:
- WO-A1-2005/123186
- US-A1- 2004 206 916
- US-A1- 2005 027 463
- US-A1- 2005 245 799
- US-A1- 2006 226 985
- US-A1- 2006 264 888
- US-A1- 2006 270 922
- US-A1- 2007 038 044
- US-A1- 2007 038 044
- US-A1- 2007 219 496
- US-A1- 2007 222 609
- US-A1- 2008 188 731
- US-B1- 6 546 268
- US-B2- 7 003 341
- US-B2- 7 134 999
- US-B2- 7 134 999

## Description

### BACKGROUND

The detection of the level of glucose or other analytes, such as lactate, oxygen or the like, in certain individuals is vitally important to their health. For example, the monitoring of glucose is particularly important to individuals with diabetes. Diabetics may need to monitor glucose levels to determine when insulin is needed to reduce glucose levels in their bodies or when additional glucose is needed to raise the level of glucose in their bodies.

Devices have been developed for continuous or automatic monitoring of analytes, such as glucose, in bodily fluid such as in the blood stream or in interstitial fluid. Some of these analyte measuring devices are configured so that at least a portion of the devices are positioned below a skin surface of a user, e.g., in a blood vessel or in the subcutaneous tissue of a user.

Ease of insertion and use, including minimal user intervention and on-body size and height (or thickness) of such transcutaneous or percutaneous medical devices that are worn on the body are important in usability, wearability, and comfort during the device usage. Moreover, for many of such medical devices that require a battery or a similar power source to perform the device specific operations, power management as well as shelf life is important.
US 2007/038044 A1 describes systems and methods for measuring an analyte in a host. US 7 134 999 B2 describes optimized sensor geometry for an implantable glucose sensor. WO 2005/123186 A1 describes a wireless communication system that includes an antenna structure adapted for coupling to a medical device antenna when the medical device is not implanted in a patient's body. US 6 546 268 B1 describes a biochemical sensor based on ball integrated circuit technology which is designed to be biocompatible for implantation within a human or animal body.

### SUMMARY

Embodiments of the subject disclosure include devices and methods and kits for providing sensor electronics assembly including an analyte sensor for monitoring of analyte levels such as glucose levels over a sensing time period. Sensing time period may be determined by the analyte sensor life, for example, including, but not limited to about three days or more, about five days or more, or about seven days or more, or about fourteen days or more.

Embodiments include methods, devices and systems for monitoring glucose levels and obtaining glucose measurements that are discreet, automated, minimally invasive and with reduced pain and repetition of glucose testing procedures to obtain multiple discrete measurements over the sensing time period. Also provided are kits.

Embodiments further include a control unit, a control command generator coupled to the control unit to receive a control signal and to generate a control command based on a carrier signal, an antenna section coupled to the control command generator to transmit the control command with the carrier signal and to receive a backscatter response data packet using the carrier signal, and a receiver section coupled to the antenna section to process the received backscatter response data packet and to generate an output glucose data.

Embodiments also include real time discrete glucose measurement data acquisition on-demand, as desired by the user or upon request, based on, for example, RFID data communication techniques for data transmission and acquisition from the analyte sensor/electronics assembly or the on-body patch device including the analyte sensor and the data processing and communication components provided in a compact, low profile housing and placed on the skin surface of the user. The analyte sensor in certain embodiments includes a portion that is transcutaneously positioned and maintained in fluid contact with an interstitial fluid under the skin surface continuously during the sensing time period as discussed above, for example.

These and other features, objects and advantages of the present disclosure will become apparent to those persons skilled in the art upon reading the details of the present disclosure as more fully described below.

### BRIEF DESCRIPTION OF THE DRAWINGS

FIG. 1 shows a data monitoring and management system such as, for example, an analyte (e.g., glucose) monitoring system in accordance with certain embodiments of the present disclosure;
FIG. 2 illustrates a data monitoring and management system for real time glucose measurement data acquisition and processing in one aspect of the present disclosure;
FIG. 3 is a block diagram of a receiver/monitor unit such as that shown in FIG. 1 in accordance with certain embodiments;
FIG. 4 is a block diagram of a reader device/receiver unit such as that shown in FIG. 2 in one aspect of the present disclosure;
FIG. 5 is an exemplary schematic of an on-body patch device including an integrated sensor and sensor electronics assembly for use in the monitoring systems of FIGS. 1 and 2 in one aspect of the present disclosure;
FIG. 6 is a block diagram of the integrated sensor and sensor electronics assembly for use in the monitoring systems of FIGS. 1 and 2 in another aspect of the present disclosure;
FIG. 7 is a schematic of the reader device/receiver unit for use in the monitoring systems of FIGS. 1 and 2 in accordance with one aspect of the present disclosure;
FIGS. 8A and 8B illustrate a top view and a side view, respectively, of antenna and electronic circuit layout of the on-body patch device including an sensor and sensor electronics assembly for use in the monitoring systems of FIGS. 1 and 2 in one aspect of the present disclosure;
FIG. 9 illustrates an exemplary circuit schematic of the on-body patch device including an sensor and sensor electronics assembly in accordance with aspects of the present disclosure;
FIG. 10A is a perspective view of the components of the on-body patch device including sensor and sensor electronics assembly in accordance with one aspect of the present disclosure;
FIG. 10B is another perspective view of the components of the on-body patch device including sensor and sensor electronics assembly in accordance with one aspect of the present disclosure;
FIG. 10C is another perspective view of the assembled on-body patch device including sensor and sensor electronics assembly in accordance with one aspect of the present disclosure;
FIGS. 11A-11C illustrate circuit layouts for the sensor electronics assembly in the on-body patch device including sensor and sensor electronics assembly in accordance with embodiments of the present disclosure;
FIGS. 12A-12B illustrate pre-deployment and post insertion configurations of the insertion device for positioning the on-body patch device including sensor and sensor electronics assembly in accordance with embodiments of the present disclosure;
FIGS. 12C-12G illustrate cross sectional perspective views of the operation of the insertion device for deploying the on-body patch device including sensor and sensor electronics assembly in accordance with embodiments of the present disclosure;
FIGS. 13A-13B illustrate embodiments of a power supply switch mechanism including conductive plugs of the on-body patch device including sensor and sensor electronics assembly in accordance with embodiments of the present disclosure;
FIGS. 13C-13E illustrate another configuration of the power supply switch mechanism including conductive pads of the on-body patch device including sensor and sensor electronics assembly in accordance with embodiments of the present disclosure;
FIG. 14 illustrates a power supply switch mechanism including an internal switch with a push rod activation of the on-body patch device including sensor and sensor electronics assembly in accordance with embodiments of the present disclosure;
FIG. 15 illustrates a power supply switch mechanism including introducer retraction trigger activation of the on-body patch device including sensor and sensor electronics assembly in accordance with embodiments of the present disclosure;
FIG. 16 illustrates a power supply switch mechanism with a contact switch of the on-body patch device including sensor and sensor electronics assembly in accordance with embodiments of the present disclosure;
FIGS. 17A-17B illustrate a power supply switch mechanism with a battery contact locking mechanism of the on-body patch device including sensor and sensor electronics assembly in accordance with embodiments of the present disclosure; and
FIGS. 18A-18B illustrate a power supply switch mechanism with a bi-modal dome switch of the on-body patch device including sensor and sensor electronics assembly in accordance with embodiments of the present disclosure.

### DETAILED DESCRIPTION

Within the scope of the present disclosure, there are provided devices, systems, kits and methods for providing compact, low profile, on-body physiological parameter monitoring device (physiological parameters such as for example, but not limited to analyte levels, temperature levels, heart rate, etc), configured for single or multiple use over a predetermined time period, which provide a low profile geometry, effective power management, improved shelf life, and ease and comfort of use including device positioning, and activation. Embodiments include an on-body assembly including a transcutaneously positioned analyte sensor and sensor electronics in a compact, low profile integrated assembly and coupled to an insertion device for deployment.

Embodiments include continuous glucose monitoring (CGM) system or routines or functions for execution operations to continuously or semi-continuously monitor an analyte level such as glucose level with the transcutaneously positioned analyte sensor, where the real time analyte measurements are provided to a data receiver unit, a reader device, a data repeater or relay device such as data processing module, a data processing terminal or a remote terminal for data processing automatically upon data sampling at predetermined time intervals or based on programmed or programmable data transmission schedule. Data processing may include display, storage, execution of related alarm or notification functions, and analysis such as generating charts or graphs based on, for example, the monitored analyte levels received from the sensor/sensor electronics assembly.

Embodiments further include analyte data acquisition in real time where the analyte level detected by the transcutaneously positioned analyte sensor is stored either permanently or temporarily in a memory or storage unit of a data processing unit or an integrated sensor and data processing unit assembly, such as an on-body patch device (stored for example, for about one day or less, or for about 10 hours or less, or for about 5 hours or less, or for about 3 hours or less, or for about one hour or less). In such embodiments, the receiver unit or the reader device may be used to acquire the detected analyte level in real time, and/or on-demand or upon request using, for example, RFID communication protocol or other suitable data communication protocols. Sampled analyte related data in certain embodiments are received by the receiver unit or the reader device upon activation or initiation by the user or the patient, for example, of a switch or other initiation mechanism to initiate the data transfer or provide data request command. Such activation switch or mechanism may be provided or included in the user interface of the reader device or the receiver unit.

Embodiments of the present disclosure relate to methods and devices for detecting at least one analyte such as glucose in body fluid. Embodiments include glucose measurements by an on-body patch device that includes a transcutaneously positioned analyte sensor in fluid contact with the body fluid such as interstitial fluid, and sensor electronics in signal communication with the analyte sensor, where the on-body patch device is configured to transmit one or more signals or data packets associated with a monitored analyte level upon detection of a reader device or the receiver unit of the analyte monitoring system within a predetermined proximity for a period of time (for example, about 10 seconds or less, or preferably about 5 seconds or less, or preferably about 2 seconds or less, or until a confirmation, such as an audible notification, is output on the reader device/receiver unit indicating successful acquisition of the analyte related signal from the on-body patch device).

For example, in one aspect, when a reader device/receiver unit is positioned within approximately 12.7cm (5 inches) or less (or about 25.4cm (10 inches) or less, for example) to the on-body patch device that is adhesively placed or mounted on the skin surface of a patient (with the analyte sensor transcutaneously positioned in fluid contact under the skin surface and in signal communication with the sensor electronics of the on-body patch device), a radio frequency source within the reader device/receiver unit may be configured to provide RF power to the on-body patch device. In response, the on-body patch device in one embodiment may be configured to generate an output signal (e.g., an RF signal) and transmit it to the reader device/receiver unit which includes, among others data indicating the glucose measurement. In one aspect the signal communication and/or RF power transmission may initiate automatically upon detection of the reader device/receiver unit within a predetermined proximity to the on-demand patch device, or alternatively the reader device/receiver unit may require a user activation or confirmation prior to initiating signal communication and/or RF power transmission with the on-body patch device as discussed above.

In a further aspect, the transmitted data from the on-body patch device to the reader device/receiver unit may include glucose trend information that was stored in the on-body patch device for a predetermined time period, since the initialization of the sensor and positioning it in fluid contact with the interstitial fluid, or since the last transmission of data to the reader device, or any one or more combinations of the above. For example, the trend information may indicate the variation in the monitored glucose level over the particular time period based on signals received from the analyte sensor and stored in the on-body patch device.

As described in further detail below, the on-body patch device may optionally include an output component such as a speaker, a light indicator (for example, an LED indicator), or the like to provide one or more indications associated with its functions such as a successful transmission of data to the reader device or the receiver unit, alarm or alert conditions associated with its internal components, or a detection of the RF power received from the reader device or the receiver unit, for example. By way of a non-limiting example, one or more exemplary output indication may include an audible sound (including for example, a short tone, a changing tone, multi-tone, one or more programmed ringtones or one or more combinations thereof), a visual indication such as a blinking light of the LED indicator, a solid light on the LED indicator maintained at a predetermined or programmed or programmable time period (for example, 5 seconds), each of which may be pre-programmed in the on-body patch device, or alternatively programmable by the user through the user interface of the reader device/receiver unit when in communication with the on-body patch device.

In a further aspect, when an alarm or alert condition is detected (for example, a detected glucose level monitored by the analyte sensor that is outside a predetermined acceptable range indicating a physiological condition which requires attention or intervention for medical treatment or analysis (for example, a hypoglycemic condition, a hyperglycemic condition, an impending hyperglycemic condition or an impending hypoglycemic condition)), the one or more output indications may be generated in the on-body patch device and presented to the patient or the user so that corrective action may be timely taken. Alternatively, the output indications may be additionally or alternatively presented or output on the reader device/receiver unit when, for example, the reader device/receiver unit is within range of the on-body patch device

In certain aspects, future or anticipated analyte levels may be predicted based on information obtained, e.g., the current analyte level, the rate of change of the analyte level and analyte trend information. Predictive alarms may be programmed or programmable in the reader device/receiver unit, or the on-body patch device, or both, and may be configured to notify the user of a predicted analyte levels that may be of concern in advance of the user's analyte level reaching the future level. This provides the user an opportunity to take corrective action.

Before the present disclosure is described in additional detail, it is to be understood that this disclosure is not limited to particular embodiments described, as such may, of course, vary. It is also to be understood that the terminology used herein is for the purpose of describing particular embodiments only, and is not intended to be limiting, since the scope of the present disclosure will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the disclosure. The upper and lower limits of these smaller ranges may independently be included in the smaller ranges is also encompassed within the disclosure, subject to any specifically excluded limit in the stated range. Where the stated range includes one or both of the limits, ranges excluding either or both of those included limits are also included in the disclosure.

Unless defined otherwise, all technical and scientific terms used herein have the same meaning as commonly understood by one of ordinary skill in the art to which this disclosure belongs. Although any methods and materials similar or equivalent to those described herein can also be used in the practice or testing of the present disclosure, the preferred methods and materials are now described.

It must be noted that as used herein and in the appended claims, the singular forms "a", "an", and "the" include plural referents unless the context clearly dictates otherwise.

The publications discussed herein are provided solely for their disclosure prior to the filing date of the present application. Nothing herein is to be construed as an admission that the present disclosure is not entitled to antedate such publication by virtue of prior disclosure. Further, the dates of publication provided may be different from the actual publication dates which may need to be independently confirmed.

As will be apparent to those of skill in the art upon reading this disclosure, each of the individual embodiments described and illustrated herein has discrete components and features which may be readily separated from or combined with the features of any of the other several embodiments without departing from the scope of the present disclosure.

The figures shown herein are not necessarily drawn to scale, with some components and features being exaggerated for clarity.

Generally, embodiments of the present disclosure relate to methods and devices for detecting at least one analyte such as glucose in body fluid. In certain embodiments, the present disclosure relates to the continuous and/or automatic in vivo monitoring of the level of an analyte using an analyte sensor.

Accordingly, embodiments include analyte monitoring devices and systems that include an analyte sensor- at least a portion of which is positionable beneath the skin of the user - for the in vivo detection, of an analyte, such as glucose, lactate, and the like, in a body fluid. Embodiments include wholly implantable analyte sensors and analyte sensors in which only a portion of the sensor is positioned under the skin and a portion of the sensor resides above the skin, e.g., for contact to a transmitter, receiver, transceiver, processor, etc. The sensor may be, for example, subcutaneously positionable in a patient for the continuous or periodic monitoring of a level of an analyte in a patient's interstitial fluid.

For the purposes of this description, continuous monitoring and periodic monitoring will be used interchangeably, unless noted otherwise. Discrete monitoring as used herein includes the acquisition or reception of monitored analyte data where real time monitored analyte level information is received or acquired on demand or in response to a request to the on-body patch device including sensor and sensor electronics. That is, embodiments include analyte sensors and sensor electronics which sample and process analyte related information based on a programmed or programmable schedule such as every minute, every five minutes and so on. Such analyte monitoring routines may be reported or transmitted in real time to the receiver unit/reader device at the time of data sampling and processing. Alternatively, as discussed, the continuously sampled analyte data and processed analyte related signals may be stored and transmitted to a remote location such as the receiver unit, data processing module, the data processing terminal, the reader device or the remote terminal in response to a request for such information from the remote location. The analyte level may be correlated and/or converted to analyte levels in blood or other fluids. In certain embodiments, an analyte sensor may be positioned in contact with interstitial fluid to detect the level of glucose, which detected glucose may be used to infer the glucose level in the patient's bloodstream. Analyte sensors may be insertable into a vein, artery, or other portion of the body containing fluid. Embodiments of the analyte sensors of the subject disclosure may be configured for monitoring the level of the analyte over a time period which may range from minutes, hours, days, weeks, or longer.

Of interest are analyte sensors, such as glucose sensors, that are capable of *in vivo* detection of an analyte for about one hour or more, e.g., about a few hours or more, e.g., about a few days of more, e.g., about three or more days, e.g., about five days or more, e.g., about seven days or more, e.g., about several weeks or at least one month. Future analyte levels may be predicted based on information obtained, e.g., the current analyte level at time t₀, the rate of change of the analyte, etc. Predictive alarms may notify the user of predicted analyte levels that may be of concern prior in advance of the analyte level reaching the future level. This enables the user an opportunity to take corrective action. Embodiments include transmission of the acquired real time analyte information on-demand from the user (using for example, the reader device/receiver unit positioned in close proximity to the low profile on-body patch device), storage of the acquired real time analyte information, and subsequent transmission based on retrieval from the storage device (such as a memory device).

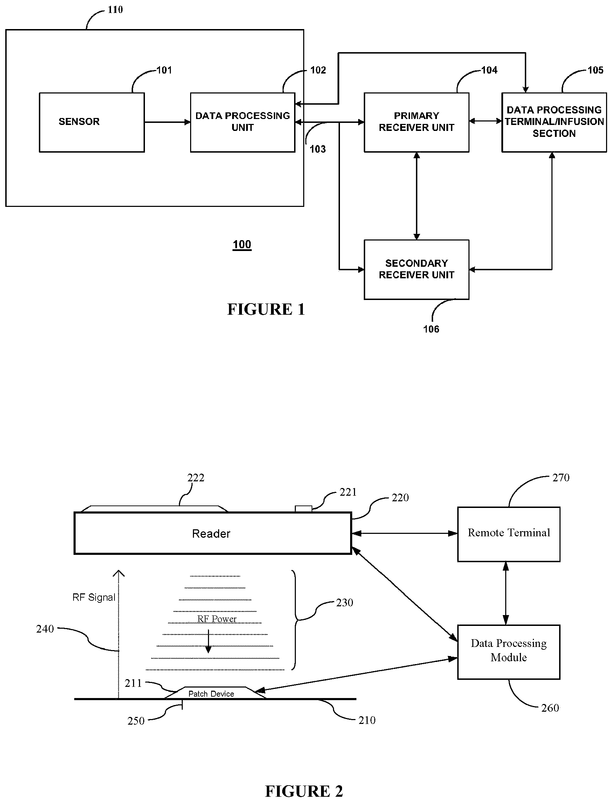

FIG. 1 shows a data monitoring and management system such as, for example, an analyte (e.g., glucose) monitoring system in accordance with certain embodiments of the present disclosure. Embodiments of the subject disclosure are described primarily with respect to glucose monitoring devices and systems, and methods of glucose detection, for convenience only and such description is in no way intended to limit the scope of the disclosure. It is to be understood that the analyte monitoring system may be configured to monitor a variety of analytes at the same time or at different times.

Analytes that may be monitored include, but are not limited to, acetyl choline, amylase, bilirubin, cholesterol, chorionic gonadotropin, creatine kinase (e.g., CK-MB), creatine, DNA, fructosamine, glucose, glutamine, growth hormones, hormones, ketones, lactate, peroxide, prostate-specific antigen, prothrombin, RNA, thyroid stimulating hormone, and troponin. The concentration of drugs, such as, for example, antibiotics (e.g., gentamicin, vancomycin, and the like), digitoxin, digoxin, drugs of abuse, theophylline, and warfarin, may also be monitored. In those embodiments that monitor more than one analyte, the analytes may be monitored at the same or different times.

Referring to FIG. 1, the analyte monitoring system 100 includes a sensor 101, a data processing unit (e.g., sensor electronics) 102 connectable to the sensor 101, and a primary receiver unit 104 which is configured to communicate with the data processing unit 102 via a communication link 103. In aspects of the present disclosure, the sensor 101 and the data processing unit (sensor electronics) 102 may be configured as a single integrated assembly 110. In certain embodiments, the integrated sensor and sensor electronics assembly 110 may be configured as an on-body patch device. In such embodiments, the on-body patch device may be configured for, for example, RFID or RF communication with a reader device/receiver unit.

In certain embodiments, the primary receiver unit 104 may be further configured to transmit data to a data processing terminal 105 to evaluate or otherwise process or format data received by the primary receiver unit 104. The data processing terminal 105 may be configured to receive data directly from the data processing unit 102 via a communication link which may optionally be configured for bi-directional communication. Further, the data processing unit 102 may include a transmitter or a transceiver to transmit and/or receive data to and/or from the primary receiver unit 104, the data processing terminal 105 or optionally the secondary receiver unit 106.

Also shown in FIG. 1 is an optional secondary receiver unit 106 which is operatively coupled to the communication link and configured to receive data transmitted from the data processing unit 102. The secondary receiver unit 106 may be configured to communicate with the primary receiver unit 104, as well as the data processing terminal 105. The secondary receiver unit 106 may be configured for bi-directional wireless communication with each of the primary receiver unit 104 and the data processing terminal 105. As discussed in further detail below, in certain embodiments the secondary receiver unit 106 may be a de-featured receiver as compared to the primary receiver unit 104, i.e., the secondary receiver unit 106 may include a limited or minimal number of functions and features as compared with the primary receiver unit 104. As such, the secondary receiver unit 106 may include a smaller (in one or more, including all, dimensions), compact housing or embodied in a device such as a wrist watch, arm band, etc., for example. Alternatively, the secondary receiver unit 106 may be configured with the same or substantially similar functions and features as the primary receiver unit 104. The secondary receiver unit 106 may include a docking portion to be mated with a docking cradle unit for placement by, e.g., the bedside for night time monitoring, and/or bi-directional communication device.

Only one sensor 101, data processing unit 102 and data processing terminal 105 are shown in the embodiment of the analyte monitoring system 100 illustrated in FIG. 1. However, it will be appreciated by one of ordinary skill in the art that the analyte monitoring system 100 may include more than one sensor 101 and/or more than one data processing unit 102, and/or more than one data processing terminal 105. Multiple sensors may be positioned in a patient for analyte monitoring at the same or different times. In certain embodiments, analyte information obtained by a first positioned sensor may be employed as a comparison to analyte information obtained by a second sensor. This may be useful to confirm or validate analyte information obtained from one or both of the sensors. Such redundancy may be useful if analyte information is contemplated in critical therapy-related decisions. In certain embodiments, a first sensor may be used to calibrate a second sensor.

The analyte monitoring system 100 may be a continuous monitoring system, or semi-continuous, or a discrete monitoring system. In a multi-component environment, each component may be configured to be uniquely identified by one or more of the other components in the system so that communication conflict may be readily resolved between the various components within the analyte monitoring system 100. For example, unique IDs, communication channels, and the like, may be used.

In certain embodiments, the sensor 101 is physically positioned in or on the body of a user whose analyte level is being monitored. The sensor 101 may be configured to at least periodically sample the analyte level of the user and convert the sampled analyte level into a corresponding signal for transmission by the data processing unit 102.

The data processing unit 102 is coupleable to the sensor 101 so that both devices are positioned in or on the user's body, with at least a portion of the analyte sensor 101 positioned transcutaneously. The data processing unit 102 in certain embodiments may include a portion of the sensor 101 (proximal section of the sensor in electrical communication with the data processing unit 102) which is encapsulated within or on the printed circuit board of the data processing unit 102 with, for example, potting material or other protective material. The data processing unit 102 performs data processing functions, where such functions may include but are not limited to, filtering and encoding of data signals, each of which corresponds to a sampled analyte level of the user, for transmission to the primary receiver unit 104 via the communication link 103. In one embodiment, the sensor 101 or the data processing unit 102 or a combined sensor/data processing unit may be wholly implantable under the skin layer of the user.

In one aspect, the primary receiver unit 104 may include an analog interface section including an RF receiver and an antenna that is configured to communicate with the data processing unit 102 via the communication link 103, and a data processing section for processing the received data from the data processing unit 102 such as data decoding, error detection and correction, data clock generation, and/or data bit recovery.

In operation, the primary receiver unit 104 in certain embodiments is configured to synchronize with the data processing unit 102 to uniquely identify the data processing unit 102, based on, for example, an identification information of the data processing unit 102, and thereafter, to periodically receive signals transmitted from the data processing unit 102 associated with the monitored analyte levels detected by the sensor 101. That is, when operating in the CGM mode, the receiver unit 104 in certain embodiments is configured to automatically receive time spaced analyte related data packets from the analyte sensor/sensor electronics when the communication link (e.g., RF range) is maintained between these components.

Referring again to FIG. 1, the data processing terminal 105 may include a personal computer, a portable data processing devices or computers such as a laptop computer or a handheld device (e.g., personal digital assistants (PDAs), communication devices such as a cellular phone (e.g., a multimedia and Internet-enabled mobile phone such as an iPhone, a Blackberry device, a Palm device such as Palm Pre, Treo, or similar phone), mp3 player, pager, and the like), drug delivery device, each of which may be configured for data communication with the receiver via a wired or a wireless connection. Additionally, the data processing terminal 105 may further be connected to a data network (not shown) for storing, retrieving, updating, and/or analyzing data corresponding to the detected analyte level of the user.

The data processing terminal 105 may include an infusion device such as an insulin infusion pump or the like, which may be configured to administer insulin to patients, and which may be configured to communicate with the primary receiver unit 104 for receiving, among others, the measured analyte level. Alternatively, the primary receiver unit 104 may be configured to integrate an infusion device therein so that the primary receiver unit 104 is configured to administer insulin (or other appropriate drug) therapy to patients, for example, for administering and modifying basal profiles, as well as for determining appropriate boluses for administration based on, among others, the detected analyte levels received from the data processing unit 102. An infusion device may be an external device or an internal device (wholly implantable in a user).

In particular embodiments, the data processing terminal 105, which may include an insulin pump, may be configured to receive the analyte signals from the data processing unit 102, and thus, incorporate the functions of the primary receiver unit 104 including data processing for managing the patient's insulin therapy and analyte monitoring. In certain embodiments, the communication link 103 as well as one or more of the other communication interfaces shown in FIG. 1 may use one or more of an RF communication protocol, an infrared communication protocol, a Bluetooth enabled communication protocol, an 802.11x wireless communication protocol, or an equivalent wireless communication protocol which would allow secure, wireless communication of several units (for example, per HIPPA requirements) while avoiding potential data collision and interference.

As described in aspects of the present disclosure, the analyte monitoring system may include an on-body patch device with a thin profile that can be worn on the arm or other locations on the body (and under clothing worn by the user or the patient), the on-body patch device including an analyte sensor and circuitry and components for operating the sensor and processing and storing signals received from the sensor as well as for communication with the reader device. For example, one aspect of the on-body patch device may include electronics to sample the voltage signal received from the analyte sensor in fluid contact with the body fluid, and to process the sampled voltage signals into the corresponding glucose values and/or store the sampled voltage signal as raw data.

In certain embodiments, the on-body patch device includes an antenna such as a loop antenna to receive RF power from the an external device such as the reader device/receiver unit described above, electronics to convert the RF power received via the antenna into DC (direct current) power for the on-body patch device circuitry, communication module or electronics to detect commands received from the reader device, and communication component to transmit data to the reader device, a low capacity battery for providing power to sensor sampling circuitry (for example, the analog front end circuitry of the on-body patch device in signal communication with the analyte sensor), one or more non-volatile memory or storage device to store data including raw signals from the sensor or processed data based on the raw sensor signals. More specifically, in the on operation demand mode, the on body patch device in certain embodiments is configured to transmit real time analyte related data and/or stored historical analyte related data when within the RF power range of the reader device. As such, when the reader device is removed of positioned out of range relative to the on body patch device, the on body patch device may no longer transmit the analyte related data.

In certain embodiments, a data processing module/terminal may be provided in the analyte monitoring system that is configured to operate as a data logger, interacting or communicating with the on-body patch device by, for example, transmitting requests for analyte level information to the on-body patch device, and storing the responsive analyte level information received from the on-body patch device in one or more memory components of the data processing module.
Further, data processing module may be configured as a compact on-body relay device to relay or retransmit the received analyte level information from the onbody patch device to the reader device/receiver unit or the remote terminal or both.
The data processing module in one aspect may be physically coupled to the onbody patch device, for example, on a single adhesive patch on the skin surface of the patient. Alternatively, the data processing module may be positioned close to but not in contact with the on-body patch device. For example, when the on-body patch device is positioned on the abdomen of the patient, the data processing module may be worn on a belt of the patient or the user, such that the desired close proximity or predetermined distance of approximately 2.54-12.7cm (1-5 inches) (or about 2.54-25.4cm (1-10 inches), for example, or more) between the on-body patch device and the data processing module may be maintained.

The various processes described above including the processes operating in the software application execution environment in the analyte monitoring system including the on-body patch device, the reader device, data processing module and/or the remote terminal performing one or more routines described above may be embodied as computer programs developed using an object oriented language that allows the modeling of complex systems with modular objects to create abstractions that are representative of real world, physical objects and their interrelationships. The software required to carry out the inventive process, which may be stored in a memory or storage device of the storage unit of the various components of the analyte monitoring system described above in conjunction to the Figures including the on-body patch device, the reader device, the data processing module, various described communication devices, or the remote terminal may be developed by a person of ordinary skill in the art and may include one or more computer program products.

In one embodiment, an apparatus for bi-directional communication with an analyte monitoring system may comprise a storage device having stored therein one or more routines, a processing unit operatively coupled to the storage device and configured to retrieve the stored one or more routines for execution, a data transmission component operatively coupled to the processing unit and configured to transmit data based at least in part on the one or more routines executed by the processing unit, and a data reception component operatively coupled to the processing unit and configured to receive analyte related data from a remote location and to store the received analyte related data in the storage device for retransmission, wherein the data transmission component is programmed to transmit a query to a remote location, and further wherein the data reception component receives the analyte related data from the remote location in response to the transmitted query when one or more electronics in the remote location transitions from an inactive state to an active state upon detection of the query from the data transmission component.

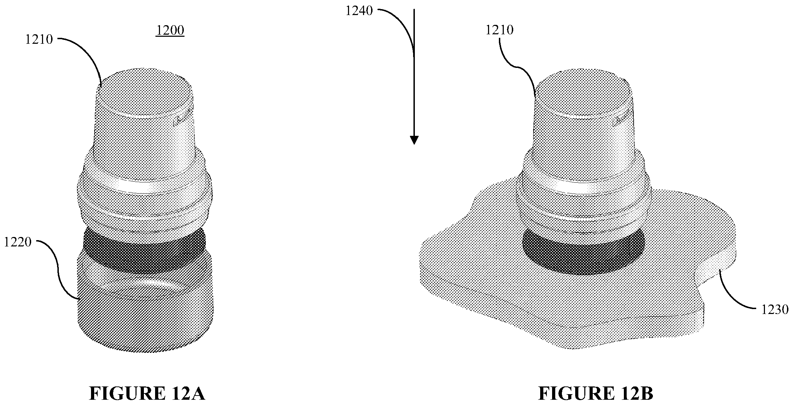

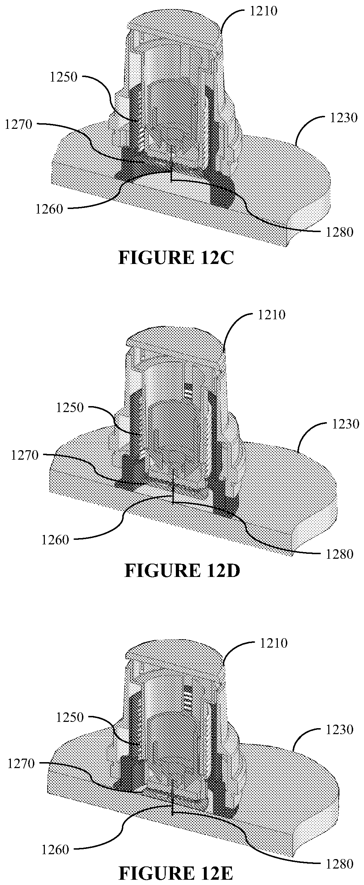

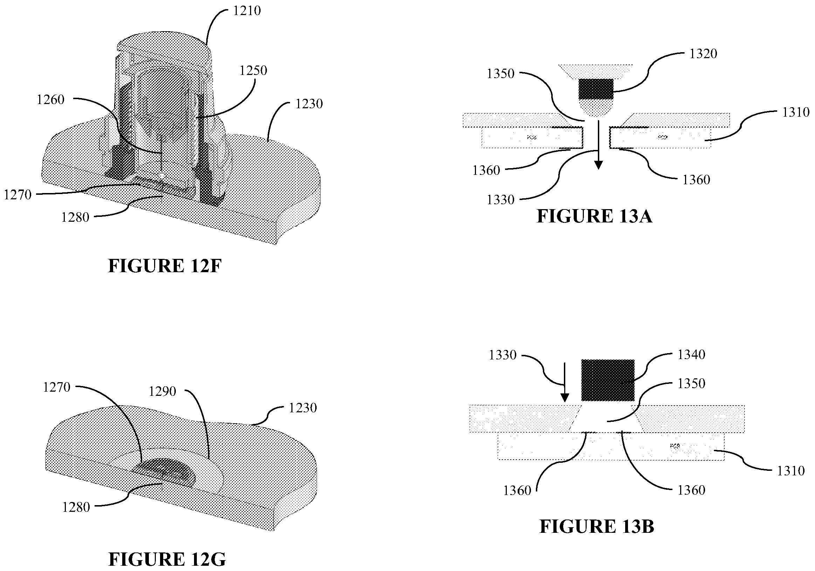

FIG. 2 illustrates a data monitoring and management system for real time glucose measurement data acquisition and processing in one aspect of the present disclosure. More specifically, as shown in FIG. 2, the on-body patch device 211 including sensor electronics coupled to an analyte sensor 250 is positioned on a skin surface 210 of a patient or a user. In one aspect, an introducer mechanism may be provided, as discussed in further detail below in conjunction with FIGS. 12A-12G, for the transcutaneous placement of the analyte sensor 250 such that when the on-body patch device 211 is positioned on the skin surface, a portion of the sensor 250 is inserted through the skin surface and in fluid contact with a body fluid of the patient or the user under the skin layer 210.

The introducer mechanism may be fully or partially automated, for example with a trigger mechanism, or may be fully or partially manual such that the sensor 250 is positioned transcutaneously by a manual operation of the user. That is, in one aspect, the on-body patch device 211 may include an introducer needle and/or lumen (and/or catheter) which may guide the sensor 250 during the insertion process through the skin layer 210. In a further aspect, the placement of the on-body patch device 211 on the skin layer 210 includes the initial piercing of the skin layer 210 with a force applied on the on-body patch device 211 in conjunction with the on-body patch device 211 placement on the skin layer 210, effectively driving the sensor 250 (and/or the introducer) through the skin layer 210. Within the scope of the present disclosure, a mechanism (such as a spring, for example) may be provided within the on-body patch device 211 or alternatively, in the introducer in cooperation with the on-body patch device 211, to withdraw the introducer needle after the sensor 250 has been positioned in fluid contact with the body fluid. In certain other embodiments, a lumen may be provided, with the analyte sensor 250 provided within the hollow cavity of the lumen for insertion, and maintained in position with the on-body patch device 211 during the time period that the on-body patch device 211 is worn on the skin layer 210.

Referring back to FIG. 2, as shown, when the reader device/receiver unit 220 is positioned or placed in close proximity and within a predetermined range of the on-body patch device 211, the RF power supply in the reader device/receiver unit 220 may be configured to provide the necessary power to operate the electronics in the on-body patch device 211, and the on-body patch device 211 may be configured to, upon detection or the RF power from the reader device/receiver unit 220, perform preprogrammed routines including, for example, transmitting one or more signals 240 to the reader device/receiver unit 220 indicative of the sampled analyte level measured by the analyte sensor 250.

In certain embodiments, the reader device/receiver unit 220 may include an RF power switch that is user activatable or activated upon positioning within a predetermined distance from the on body patch device 211 to turn on the analyte sensor in the on body patch device 211. That is, using the RF signal, the analyte sensor coupled to the sensor electronics in the on-body patch device 211 may be initialized or activated. In another embodiment, a passive RFID function may be provided or programmed such that upon receiving a "turn on" signal which, when authenticated, will turn on the electronic power switch that activates the on-body patch device 211. That is, the passive RFID configuration may include drawing energy from the RF field radiated from the reader device/receiver unit 220 so as to prompt for and/or detect the "turn on" signal which, upon authentication, activates the on body patch device 211.

In one embodiment, communication and/or RF power transfer between the reader device/receiver unit 220 and the on-body patch device 211 may be automatically initiated when the reader device/receiver unit 220 is placed in close proximity to the on-body patch device 211 as discussed above. Alternatively, the reader device/receiver unit 220 may be configured such that user activation, such as data request initiation and subsequent confirmation by the user using, for example, the display 222 and/or input components 221 of the reader device/receiver unit 220, may be required prior to the initiation of communication and/or RF power transfer between the reader device/receiver unit 220 and the on-body patch device 211. In a further embodiment, the reader device/receiver unit 220 may be user configurable between multiple modes, such that the user may choose whether the communication between the reader device/receiver unit 220 and on-body patch device 211 is performed automatically or requires a user activation and/or confirmation.

As further shown in FIG. 2, the reader device/receiver unit 220 may include display 222 or output component to provide output indication to the user or the patient, including, for example, the corresponding glucose level measurement. The display 222 of the reader device/receiver unit 220 may be additionally configured to provide the functionalities of a user interface to present other information such as alarm or alert notification to the user. In one aspect, the reader device/receiver unit 220 may include other output components such as a speaker, vibratory output component and the like to provide audible and/or vibratory output indication to the user in addition to the visual output indication provided on the display 222. Moreover, the reader device/receiver unit 220 may also include one or more input components 221 (such as, for example, push buttons, switches, capacitive sliders, jog wheels, etc.) for receiving input commands or information from the user or the patient by operation of the input components 221. In one embodiment, the display 222 and the input component 221 may be integrated into a single component, for example as a touch screen display. In such an embodiment, the user may be able to manipulate the reader device/receiver unit 220 by utilizing a set of pre-programmed motion commands, including, but not limited to, single or double tapping the display, dragging a finger or instrument across the display, motioning multiple fingers toward one another, motioning multiple fingers away from one another, etc. Other embodiments include the use of "soft buttons", whereby the input components 221 correspond to dynamic menus on the display 222 to control features and operation of the reader device/receiver unit 220. In yet another embodiment, the input component 221 may include a microphone and the reader device/receiver unit 220 may include software configured to analyze audio input received from the microphone, such that functions and operation of the reader device/receiver unit 220 may be controlled audibly by the user or patient.

As discussed, some or all of the electronics in the on-body patch device 211 in one embodiment may be configured to rely on the RF power received from the reader device/receiver unit 220 to perform analyte data processing and/or transmission of the processed analyte information to the reader device/receiver unit 220. That is, the on-body patch device 211 may be discreetly worn on the body of the user or the patient, and under clothing, for example, and when desired, by positioning the reader device/receiver unit 220 within a predetermined distance from the on-body patch device 211, real time glucose level information may be received by the reader device/receiver unit 220. This routine may be repeated as desired by the patient (or on-demand, for example) to determine glucose levels at any time during the time period that the on-body patch device 211 is worn by the user or the patient.

Referring still to FIG. 2, also shown are a data processing module/terminal 260 and a remote terminal 270. In one aspect, data processing module 260 may include a stand alone device configured for bi-directional communication to communicate with the on-body patch device 211, the reader device/receiver unit 220 and/or the remote terminal 270. More specifically, data processing module 260 may include one or more microprocessors or similar data processing components configured to execute one or more software routines for communication, as well as data storage and retrieval to and from one or more memory components provided in the housing of the data processing module 260.

The data processing module 260 in one embodiment may be configured to communicate with the on-body patch device 211 in a similar manner as the reader device/receiver unit 220 and may include communication components such as antenna, power supply and memory, among others, for example, to allow provision of RF power to the on-body patch device 211 or to request or prompt the on-body patch device 211 to send the current analyte related data and optionally other stored analyte related data. The data processing module 260 may be configured to interact with the on-body patch device 211 in a similar manner as the reader device/receiver unit 220 such that the data processing module 260 may be positioned within a predetermined distance from the on-body patch device 211 for communication with the on-body patch device 211.

In one aspect, the on-body patch device 211 and the data processing module 260 may be positioned on the skin surface of the user or the patient within the predetermined distance of each other (for example, within approximately 12.7cm (5 inches) or less) such that the communication between the on-body patch device 211 and the data processing module 260 is maintained. In a further aspect, the housing of the data processing module 260 may be configured to couple to or cooperate with the housing of the on-body patch device 211 such that the two devices are combined or integrated as a single assembly and positioned on the skin surface.

Referring again to FIG. 2, the data processing module 260 may be configured or programmed to prompt or ping the on-body patch device 211 at a predetermined time interval such as once every minute, or once every five minutes or once every 30 minutes or any other suitable or desired programmable time interval to request analyte related data from the on-body patch device 211 which is received and is stored in one or more memory devices or components of the data processing module 260. In another embodiment, the data processing module 260 is configured to prompt or ping the on-body patch device 211 when desired by the patient or the user on-demand, and not based on a predetermined time interval. In yet another embodiment, the data processing module 260 is configured to prompt or ping the on-body patch device 211 when desired by the patient or the user upon request only after a programmable time interval has elapsed. For example, in certain embodiments, if the user does not initiate communication within a programmed time period, such as, for example 5 hours from last communication (or 10 hours from the last communication), the data processing module 260 may by programmed to automatically ping or prompt the on-body patch device 211 or alternatively, initiate an alarm function to notify the user that an extended period of time has elapsed since the last communication between the data processing module 260 and the on-body patch device 211. In this manner, users, healthcare providers, or the patient may program or configure the data processing module 260 to provide certain compliance with analyte monitoring regimen, so that frequent determination of analyte levels is maintained or performed by the user. Similar functionalities may be provided or programmed in the receiver unit or the reader device in certain embodiments.

As further shown in FIG. 2, the data processing module 260 in one aspect may be configured to transmit the stored data received from the on-body patch device 211 to the reader device/receiver unit 220 when communication between the data processing module 260 and the reader device/receiver unit 220 is established. More specifically, in addition to RF antenna and RF communication components described above, data processing module 260 may include components to communicate using one or more wireless communication protocols such as, for example, but not limited to, infrared (IR) protocol, Bluetooth protocol, Zigbee protocol, and 802.11 wireless LAN protocol. Additional description of communication protocols including those based on Bluetooth protocol and/or Zigbee protocol can be found in U.S. Patent Publication No. 2006/0193375. The data processing module 260
may further include communication ports, drivers or connectors to establish wired communication with one or more of the reader device/receiver unit 220, on-body patch device 211, or the remote terminal 270 including, for example, but not limited to USB connector and/or USB port, Ethernet connector and/or port, Fire Wire connector and/or port, or RS-232 port and/or connector.

In one aspect, the data processing module 260 may be configured to operate as a data logger configured or programmed to periodically request or prompt the on-body patch device 211 to transmit the analyte related information, and to store the received information for later retrieval or subsequent transmission to the reader device/receiver unit 220 or to the remote terminal 270 or both, for further processing and analysis. Further, the memory or storage component in the data processing module 260 may be sufficiently large to store or retain analyte level information over an extended time period, for example, coinciding with the usage life of the analyte sensor 250 in the on-body patch device 211. In this manner, the analyte monitoring system described above in conjunction with FIGS. 1 and 2 may be configured to operate in a CGM (continuous glucose monitoring) mode such that a continuous, time spaced monitored analyte level may be received from the on-body patch device 211 and stored in the data processing module 260. The stored data in the data processing module 260 may be subsequently provided to or transmitted to the reader device/receiver unit 220, the remote terminal 270 or the like for further analysis such as identifying frequency of periods of glycemic level excursions over the monitored time period to improve or enhance therapy related decisions. Using this information, the doctor, healthcare provider or the patient may adjust or recommend modification to the diet, daily habits and routines such as exercise, and the like.

In a further aspect, the functionalities of the data processing module 260 may be configured or incorporated into a memory device such as an SD card, microSD card, compact flash card, XD card, Memory Stick card, Memory Stick Duo card, or USB memory stick/device including software programming resident in such devices to execute upon connection to the respective one or more of the on-body patch device 211, the remote terminal 270 or the reader device/receiver unit 220. In a further aspect, the functionalities of the data processing module 260, including executable software and programming, may be provided to a communication device such as a mobile telephone including, for example, iPhone, iTouch, Blackberry device, Palm based device (such as Palm Pre, Treo, Treo Pro, Centro), personal digital assistants (PDAs) or any other communication enabled operating system (such as Windows or Android operating systems) based mobile telephones as a downloadable application for execution by the downloading communication device. To this end, the remote terminal 270 as shown in FIG. 2 may include a personal computer, or a server terminal that is configured to provide the executable application software to the one or more of the communication devices described above when communication between the remote terminal 270 and the devices are established. In still a further aspect, the executable downloadable application may be provided over-the-air (OTA) as an OTA download such that wired connection to the remote terminal 270 is not necessary. In this configuration, the executable application may be automatically downloaded as an available download to the communication device, and depending upon the configuration of the communication device, installed on the device for use automatically, or based on user confirmation or acknowledgement on the communication device to execute the installation of the application.

Depending upon the user setting or configuration on the communication device, the downloaded application may be programmed or customized using the user interface of the respective communication device (screen, keypad, and the like) to establish or program the desired settings such as hyperglycemia alarm, hypoglycemia alarm, sensor replacement alarm, sensor calibration alarm, or any other alarm or alert conditions as may be desired by the user. Moreover, the programmed notification settings on the communication device may be output using the output components of the respective communication devices, such as speaker, vibratory output component, or visual output/display. As a further example, the communication device may be provided with programming and application software to communicate with the on-body patch device 211 such that a frequency or periodicity of data acquisition is established. In this manner, the communication device may be configured to conveniently receive analyte level information from the on-body patch device 211 at predetermined time periods such as, for example, but not limited to once every minute, once every five minutes, or once every 10 or 15 minutes, and store the received information, as well as to provide real time display of the monitored or received analyte level information and other related output display such as trend indication of the analyte level (for example, based on the received analyte level information), projection of future analyte levels based on the analyte trend, and any other desired or appropriate warning indication or notification to the user or the patient.

Information, such as trend information, for example, may be output on one or more of the reader device/receiver unit 220, data processing module 260, remote terminal 270, or any other connected device with output capabilities. Trend, and other, information may be output on a display unit of a device, for example the display 222 of the reader device/receiver unit 220. Trend information may be displayed as, for example, a graph (such as a line graph) to indicate to the user or patient the current, historical, and predicted future analyte levels as measured and predicted by the analyte monitoring system. Trend information may also be displayed as trend arrows, indicating whether the analyte level is increasing or decreasing as well as the acceleration or deceleration of the increase or decrease in analyte level. This information may be utilized by the user or patient to determine any necessary corrective actions to ensure the analyte level remains within an acceptable and/or clinically safe range. Other visual indicators, including colors, flashing, fading, etc., as well as audio indicators including a change in pitch, volume, or tone of an audio output and/or vibratory or other tactile indicators may also be incorporated into the display of trend data as means of notifying the user or patient of the current level and/or direction and/or rate of change of the level of the monitored analyte.

Additionally, when integrated with the functionalities of the data processing module 260, the communication devices described above may be programmed to operate in the optional CGM mode to receive the time spaced monitored analyte level information from the on-body patch device 211.

Referring back to the remote terminal 270 of FIG. 2, in one aspect, software updates such as software patches, firmware updates or driver upgrades, among others, to the reader device/receiver unit 220, on-body patch device 211 or the data processing module 260 may be provided by the remote terminal 270 when communication between the remote terminal 270 and the reader device/receiver unit 220 and/or the data processing module 260 is established. In still another aspect, software upgrades, programming changes or modification to the on-body patch device 211 may be received from the remote terminal 270 by one or more of the reader device/receiver unit 220 or the data processing module 260, and thereafter, provided to the on-body patch device 211 by the reader device/receiver unit 220 or the data processing module 260.

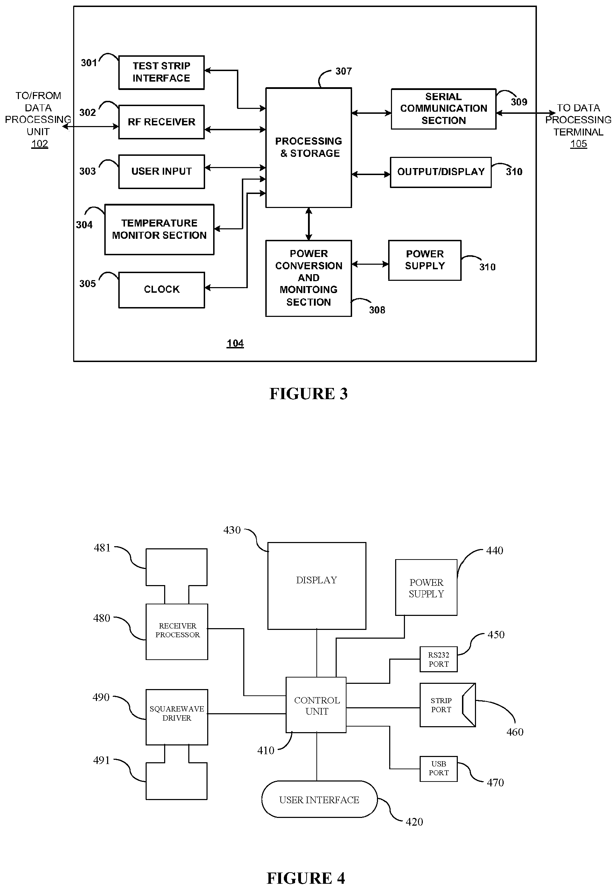

FIG. 3 is a block diagram of a receiver/monitor unit such as that shown in FIG. 1 in accordance with certain embodiments. The primary receiver unit 104 (FIG. 1) includes one or more of: a blood glucose test strip interface 301, an RF receiver 302, an input 303, a temperature detection section 304, and a clock 305, each of which is operatively coupled to a processing and storage section 307. The primary receiver unit 104 also includes a power supply 306 operatively coupled to a power conversion and monitoring section 308. Further, the power conversion and monitoring section 308 is also coupled to the receiver processor 307. Moreover, also shown are a receiver serial communication section 309, and an output 310, each operatively coupled to the processing and storage unit 307. The receiver may include user input and/or interface components or may be free of user input and/or interface components.

In certain embodiments, the test strip interface 301 includes a glucose level testing portion to receive a blood (or other body fluid sample) glucose test or information related thereto. For example, the interface may include a test strip port to receive a glucose test strip. The device may determine the glucose level of the test strip, and optionally display (or otherwise notice) the glucose level on the output 310 of the primary receiver unit 104. Any suitable test strip may be employed, e.g., test strips that only require a very small amount (e.g., one microliter or less, e.g., about 0.5 microliter or less, e.g., about 0.1 microliter or less), of applied sample to the strip in order to obtain accurate glucose information, e.g. FreeStyle® or Precision® blood glucose test strips and systems from Abbott Diabetes Care Inc. Glucose information obtained by the *in vitro* glucose testing device may be used for a variety of purposes, computations, etc. For example, the information may be used to calibrate sensor 101, confirm results of the sensor 101 to increase the confidence thereof (e.g., in instances in which information obtained by sensor 101 is employed in therapy related decisions), etc.

In one aspect, the RF receiver 302 is configured to communicate, via the communication link 103 (FIG. 1) with the data processing unit (sensor electronics) 102, to receive encoded data from the data processing unit 102 for, among others, signal mixing, demodulation, and other data processing. The input 303 of the primary receiver unit 104 is configured to allow the user to enter information into the primary receiver unit 104 as needed. In one aspect, the input 303 may include keys of a keypad, a touch-sensitive screen, and/or a voice-activated input command unit, and the like. The temperature monitor section 304 may be configured to provide temperature information of the primary receiver unit 104 to the processing and control section 307, while the clock 305 provides, among others, real time or clock information to the processing and storage section 307.

Each of the various components of the primary receiver unit 104 shown in FIG. 3 is powered by the power supply 306 (or other power supply) which, in certain embodiments, includes a battery. Furthermore, the power conversion and monitoring section 308 is configured to monitor the power usage by the various components in the primary receiver unit 104 for effective power management and may alert the user, for example, in the event of power usage which renders the primary receiver unit 104 in sub-optimal operating conditions. The serial communication section 309 in the primary receiver unit 104 is configured to provide a bi-directional communication path from the testing and/or manufacturing equipment for, among others, initialization, testing, and configuration of the primary receiver unit 104.

Serial communication section 104 can also be used to upload data to a computer, such as time-stamped blood glucose data. The communication link with an external device (not shown) can be made, for example, by cable (such as USB or serial cable), infrared (IR) or RF link. The output/display 310 of the primary receiver unit 104 is configured to provide, among others, a graphical user interface (GUI), and may include a liquid crystal display (LCD) for displaying information. Additionally, the output/display 310 may also include an integrated speaker for outputting audible signals as well as to provide vibration output as commonly found in handheld electronic devices, such as mobile telephones, pagers, etc. In certain embodiments, the primary receiver unit 104 also includes an electroluminescent lamp configured to provide backlighting to the output 310 for output visual display in dark ambient surroundings.

Referring back to FIG. 3, the primary receiver unit 104 may also include a storage section such as a programmable, non-volatile memory device as part of the processor 307, or provided separately in the primary receiver unit 104, operatively coupled to the processor 307. The processor 307 may be configured to perform Manchester decoding (or other protocol(s)) as well as error detection and correction upon the encoded data received from the data processing unit 102 via the communication link 103.

In further embodiments, the data processing unit 102 and/or the primary receiver unit 104 and/or the secondary receiver unit 105, and/or the data processing terminal/infusion section 105 may be configured to receive the blood glucose value wirelessly over a communication link from, for example, a blood glucose meter. In further embodiments, a user manipulating or using the analyte monitoring system 100 (FIG. 1) may manually input the blood glucose value using, for example, a user interface (for example, a keyboard, keypad, voice commands, and the like) incorporated in the one or more of the data processing unit 102, the primary receiver unit 104, secondary receiver unit 105, or the data processing terminal/infusion section 105.

Additional detailed descriptions are provided in U.S. Patent Nos. 5,262,035; 5,264,104; 5,262,305; 5,320,715; 5,593,852; 6,175,752; 6,650,471; 6,746, 582, 6,284,478, 7,299,082, and in application No. 10/745,878 filed December 26, 2003 (with Publication No. US 2004/0186365 A1) titled "Continuous Glucose Monitoring System and Methods of Use", and in application No. 11/060,365 filed February 16, 2005 (with Publication No. US 2005/0182306 A1) titled "Method and System for Providing Data Communication in Continuous Glucose Monitoring And Management System".

FIG. 4 is a block diagram of a reader device/receiver unit such as that shown in FIG. 2 in one aspect of the present disclosure. Referring to FIG. 4, in one aspect the reader device/receiver unit includes a control unit 410, such as one or more microprocessors, operatively coupled to a display 430 and a user interface 420. The reader device/receiver unit may also include one or more data communication ports such as USB port (or connector) 470 or RS-232 port 450 (or any other wired communication ports) for data communication with other devices such as a personal computer, a server, a mobile computing device, a mobile telephone, a pager, or other handheld data processing devices including smart phones such as Blackberry, iPhone and Palm based mobile devices, with data communication and processing capabilities including data storage and output.

Referring to FIG. 4, a power supply 440, such as one or more batteries, is also provided and operatively coupled to the control unit 410 and configured to provide the necessary power to the reader device/receiver unit for operation. In addition, referring still again to FIG. 4, the reader device/receiver unit may include a loop antenna 481 such as a 433MHz (or other equivalent) loop antenna coupled to a receiver processor 480 (which may include a 433MHz receiver chip, for example) for wireless communication with the sensor electronics in the on-body patch device/sensor data processing unit. Additionally, a primary inductive loop antenna 491 is provided and coupled to a squarewave driver 490 which is operatively coupled to the control unit 410.

Referring still to FIG. 4, the reader device/receiver unit of the analyte monitoring system may include a strip port 460 configured to receive an in vitro test strip, the strip port 460 coupled to the control unit 410, and further, where the control unit 410 includes programming to process the sample on the in vitro test strip which is received in the strip port 460. Furthermore, within the scope of the present disclosure some of the components of the reader device/receiver unit shown in FIG. 4 may be integrated as a single component such as the user interface 420 and the display 430 may be configured as a single touch sensitive display which may be configured to include soft buttons of the display itself, operable by the user or the patient for providing input commands or information to the reader device.

In one aspect, the reader device/receiver unit of the analyte monitoring system described herein may be configured to include a compact form factor, similar to a USB memory device, where the USB port 470 may be configured as a USB connector for insertion or connection to a USB port on another device such as a personal computing device or the like. Such compact form factor may include some or all of the components of the reader device/receiver unit described above.

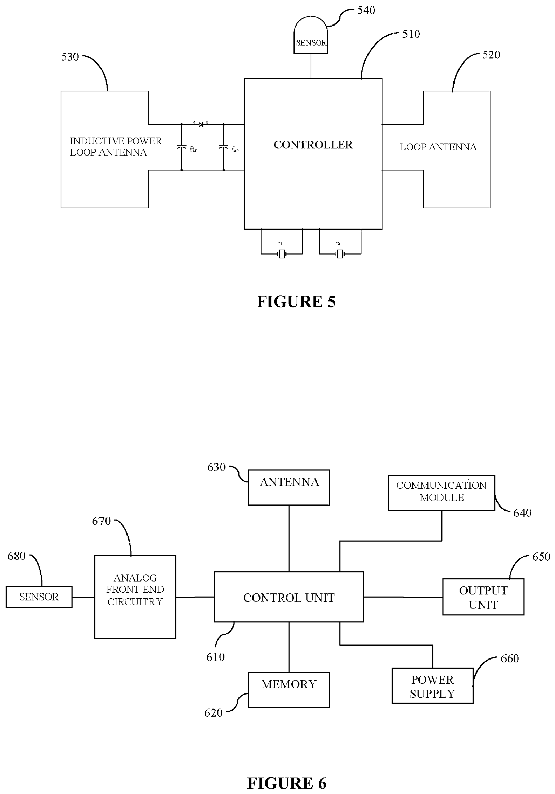

FIG. 5 is an exemplary schematic of an on-body patch device including an integrated sensor and sensor electronics assembly for use in the analyte monitoring systems of FIGS. 1 and 2 in one aspect of the present disclosure. As shown in FIG. 5, the integrated sensor and sensor electronics assembly/on-body patch device of the analyte monitoring system, in one aspect, may include a loop antenna 520 for transmitting the analyte related data to the reader device/receiver unit and further, an inductive power loop antenna 530 for processing the RF power from the reader device/receiver unit, and including converting the RF power to corresponding DC power for the operation of the electronics of the on-body patch device. In this manner, in one aspect of the present disclosure, the on-body patch device may be configured to operate as a passive data transmitter, adopting inductive coupling power without a separate power supply or battery for data transmission. Furthermore, the on-body patch device in one aspect does not require a mechanism to turn the device in operational mode nor to deactivate or turn off the on-body patch device. That is, the on-body patch device may be configured to enter an active or operational mode when it detects the RF power from the reader device. Further shown in FIG. 5 is a plurality of super capacitors C1, C2 coupled to the inductive power loop antenna 530 and the controller 510. Referring still to FIG. 5, the controller 510 may be provided on a printed circuit board assembly including the loop antenna 520, thermistor (not shown), analyte sensor contact pads for coupling to the electrodes of the sensor 540, one or more storage devices such as non-volatile memory (not shown), and other discrete components. In certain aspects, the printed circuit board assembly may be partially or fully encapsulated with, for example, potting material.

FIG. 6 is a block diagram of the integrated sensor and sensor electronics assembly for use in the analyte monitoring systems of FIGS. 1 and 2 in another aspect of the present disclosure. Referring to FIG. 6, in certain aspects of the present disclosure, the on-body patch device includes a control unit 610 (such as, for example but not limited to, one or more microprocessors, and/or application specific integrated circuits (ASICs)), operatively coupled to analog front end circuitry 670 to process signals such as raw voltage or current signals received from the sensor 680. Also shown in FIG. 6 is a memory 620 operatively coupled to the control unit 610 for storing data and/or software routines for execution by the control unit 610. That is, the control unit 610 may be configured to access the data or routines stored in the memory 620 to update, store or replace information in the memory 620, in addition to retrieving one or more stored routines for execution. Also shown in FIG. 6 is a power supply 660 which, in certain embodiments, provides power to the electronics of the on-body patch device for operation, under the control of the control unit 610, to process signals from the sensor 680 and to store the processed sensor data for subsequent transmission to the reader device/receiver unit when prompted or pinged by the reader device/receiver unit for transmission of the stored data in addition to the real time analyte level data. As discussed above, in certain embodiments, the on-body patch device does not include the power supply 660 and is configured to rely upon the RF power from the reader device.

Additionally, an optional output unit 650 is provided to the on-body patch device as shown in FIG. 6. In certain embodiments, the output unit 650 may include an LED indicator, for example, to alert the user or the patient of one or more predetermined conditions associated with the operation of the on-body patch device and/or the determined analyte level. For example, in one aspect, the on-body patch device may be programmed or configured to provide a visual indication to notify the user of one or more predetermined operational conditions of the on-body patch device. The one or more predetermined operational conditions may be configured by the user or the patient or the healthcare provider, so that certain conditions are associated with an output indication on the on-body patch device. By way of nonlimiting example, the on-body patch device may be programmed to assert a notification using the LED indicator on the on-body patch device when signals from the sensor 680 are indicated to be beyond a programmed acceptable range (based on one sampled sensor data point, or multiple sensor data points), potentially indicating a health risk condition such as hyperglycemia or hypoglycemia, or the onset of such conditions. With such prompt or indication, the user or the patient may be timely informed of such potential condition, and using the reader device, acquire the glucose level information from the on-body patch device to confirm the presence of such conditions so that timely corrective actions may be taken.

In certain embodiments, the on-body patch device may include a speaker or an audible output component instead of or in addition to the LED indicator to provide an audible indication of one or more such conditions described above. The type of audible output may be programmed or programmable in the on-body patch device, for example, via the reader device, and may include a standard audible tone (monotone or multi tone), or include one or more ring tones provided to the on-body patch device. In certain embodiments, different conditions may be associated with a different type of audible output/alert such that the patient or the user may easily recognize the underlying detected condition based on the type of audible notification. For example, different levels of audible tones may be associated (programmed by the user or the patient, or pre-programmed in the on-body patch device) with different conditions such that when asserted, each outputted tone may be easily recognized by the user or the patient as an indication of the particular associated condition. That is, the detected onset of hyperglycemic condition based on the signal from the analyte sensor may be associated with a first predetermined loudness and/or tone, while the detected onset of hypoglycemic condition based on the signal from the analyte sensor may be associated with a second predetermined loudness and/or tone. Alternatively, the programmed or programmable audible alerts may include one or more sequence of audible outputs that are output based on a temporally spaced sequence or a sequence indicating an increase or decrease in the level of loudness (using the same tone, or gradually increasing/decreasing tones).

Furthermore, in aspects of the present disclosure the audible output indication may be asserted in conjunction with the visual output indicator, simultaneously or altematingly, as may be customized or programmable in the onbody patch device or pre-programmed.

Referring again to FIG. 6, the antenna 630 and the communication module 640 operatively coupled to the control unit 610 may be configured to detect and process the RF power when in predetermined proximity to the reader device/receiver unit providing the RF power, and further, in response, to transmit the analyte level information and optionally analyte trend information based on stored analyte level data, to the reader device. In certain aspects, the trend information may includes a plurality of analyte level information over a predetermined time period that are stored in the memory 620 of the on-body patch device and provided to the reader device/receiver unit with the real time analyte level information. For example, the trend information may include a series of time spaced analyte level data for the time period since the last transmission of the analyte level information to the reader device. Alternatively, the trend information may include analyte level data for the prior 30 minutes or one hour that are stored in memory 620 and retrieved under the control of the control unit 610 for transmission to the reader device.

Referring back to the Figures, in one aspect the on-body patch device and the reader device/receiver unit may be configured to communicate using RFID (radio frequency identification) techniques where the reader device/receiver unit is configured to interrogate the on-body patch device (associated with an RFID tag) over an RF communication link, such that the on-body patch device, in response to the RF interrogation signal from the reader device, transmits an RF response signal including, for example, data associated with the sampled analyte level from the sensor. Additional information regarding the operation of RFID communication can be found in U.S. Patent Publication No. 2009/0108992 and U.S. Patent No. 7,545,272.

For example, in one embodiment, the reader device/receiver unit may include a backscatter RFID reader configured to transmit an RF field such that when the on-body patch device is within the transmitted RF field, its antenna is tuned and in tum provides a reflected or response signal (for example, a backscatter signal) to the reader device. The reflected or response signal may include sampled analyte level data from the analyte sensor.

In one aspect, the reader device/receiver unit may be configured such that when the reader device/receiver unit is positioned in close proximity to the onbody patch device and receives the response signal from the on-body patch device,
the reader device/receiver unit is configured to output an indication (audible, visual or otherwise) to confirm the analyte level measurement acquisition. That is, during the course of the 5 to 10 days of wearing the on-body patch device on the body, the user or the patient may at any time position the reader device/receiver unit within a predetermined distance (for example, approximately 2.54-12.7cm (1-5 inches)) from the on-body patch device, and after waiting a few seconds, output an audible indication confirming the receipt of the real time analyte level information. The received analyte information may be output to the display 430 (FIG. 4) of the reader device/receiver unit for presentation to the user or the patient.

As shown above, the on-body patch device is configured to be worn over a predetermined time period on the body of the user or the patient. Accordingly, certain embodiments described below include configurations of the on-body patch device to provide for a compact configuration which is configured to remain adhered to the skin surface for the predetermined wear time period comfortably and without detaching from the skin surface. For example, in one aspect, the onbody patch device may include a single integrated housing or body assembly that includes the analyte sensor, electronics and an adhesive path. Such configuration provides for fewer parts that require manipulation by the patient or the user, leading to improved ease of use, and further, with an overmolded assembly, may be configured to provide the desired water tight seal during the course of the wear, preventing moisture or other contaminants from entering into the on-body patch device housing. Such single body configurations may additionally provide ease of manufacturing with the fewer components that require assembly.

In a further aspect, the on-body patch device may include a two part assembly including a reusable electronics component mated or coupled (detachably or fixedly) to a disposable component including the analyte sensor, a base or mount for the electronics component, and the adhesive patch.

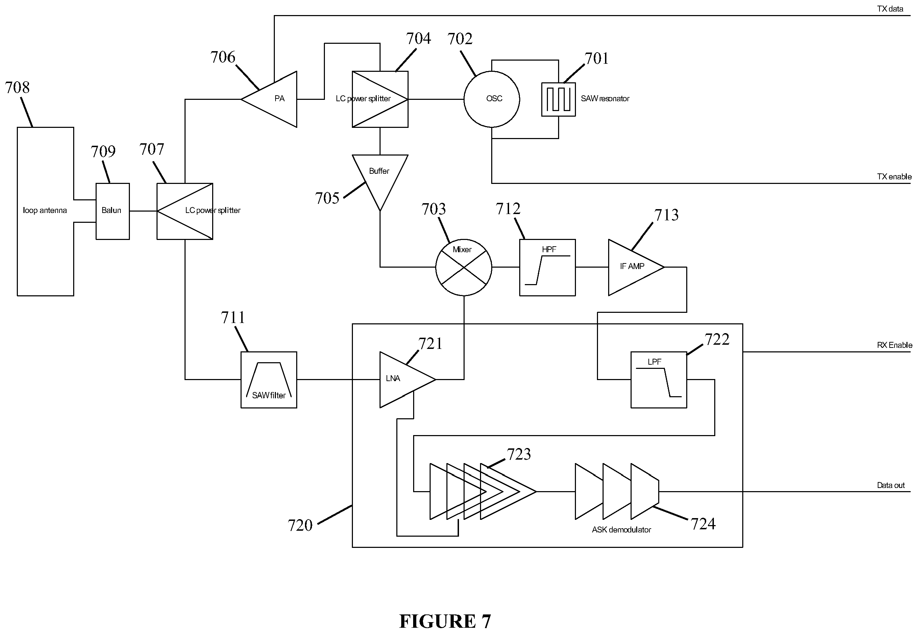

FIG. 7 is a schematic of the reader device/receiver unit for use in the analyte monitoring systems of FIGS. 1 and 2 in accordance with one aspect of the present disclosure. Referring to the Figure, the reader device/receiver unit 220 (FIG. 2) or the handheld controller in accordance with one aspect of the present disclosure, includes a surface acoustic wave (SAW) resonator 701 which may includes a resonator that generates the RF signal operating in conjunction with an oscillator (OSC) 702. The oscillator 702 is the active RF transistor component, and in conjunction with the SAW resonator 701, is configured to send out control commands (the ping signals), transmit the RF power to receive the backscatter signal from the on-body patch unit, and generate local oscillation signal to the mixer 703, as described in further detail below.

More specifically, in one aspect of the present disclosure, in operation, the transmit data (TX data) as shown is the control signal received from the control unit 410 of the reader device/receiver unit (see e.g., FIG. 4) and received from the power amplifier (PA) 706 is the RF control command to be transmitted to the on-body patch device. The SAW resonator 701 in one embodiment is configured to provide the carrier signal for the control commands (ping signals). The control signal from the control unit 410 in one embodiment include data packets that are to be transmitted to the on-body patch device to ping it to return a response signal back to the reader device.

In one embodiment, before the control signal is sent, a turn on signal from the control unit 410 is received at the TX enable line (as shown in FIG. 7) and provided to the oscillator 702. After the control signal from the control unit 410 is provided to the oscillator 702 and the SAW resonator 701, the carrier signal which is used to carry the control signal is maintained. The same carrier signal in one embodiment may be used to receive the response data packet from the on-body patch device. When the RF control signal is provided to the on-body patch device using the loop antenna and over the carrier signal, the RF power is provided at the same time (radiation energy) where the RF power is generated by the oscillator 702 in conjunction with the SAW resonator 701. In certain aspects, because the carrier signal is maintained during transmit/receive time periods between the reader device/receiver unit and the on-body patch device, the RF power is provided during the ping (or control signal) request transmission of the RF control signal and also during the time period when the backscatter response is received from the on-body patch device. In certain aspects, the reader device/receiver unit loop antenna 708 uses the same carrier signal to transmit the RF power and the RF control signal to the on-body patch device.

Referring back to FIG. 7, further shown is an LC power splitter 704 which his configured in one aspect of the present disclosure, to split the power two ways to the buffer 705 and to the power amplifier (PA) 706. The buffer 705 in one embodiment is configured to boost the RF signal received from LC power splitter 704. Output of the power amplifier 706 is the control command that is provided to a second LC power splitter 707 which splits the antenna signal (from the loop antenna into transmit signal (the control signal) and the receive signal (backscatter signal from the on-body patch device)). That is, in one embodiment, the second LC power splitter 707 may be configured to manage the transmit/receive signals using one loop antenna 708. Referring again to FIG. 7, a balun 709 provided between the loop antenna 708 and the second LC power splitter 707 is used in one embodiment to match the balanced signal from the loop antenna 708 to the unbalanced signal from the power splitter 707 (as most circuit components are unbalanced relative to ground terminal). The balun 709 includes, in one embodiment, an electrical transformer that can convert electrical signals that are balanced about ground (differential) to signals that are unbalanced (single-ended), and vice versa, using electromagnetic coupling for operation.

Referring still to FIG. 7, the loop antenna 708 transmits the RF control signal (the ping signal) and in response, receives a backscatter signal from the on-body patch device. In one aspect, the received backscatter response signal by the loop antenna is passed through the balun 709, and to the power splitter 707 to the SAW filter 711. SAW filter 711 in one aspect includes a bandpass filter configured to remove noise or interference components in the received backscatter signal, for example. The output of the SAW filter 711 is passed through ASK receiver 720. In one aspect, the ASK receiver 720 includes a low noise amplifier (LNA) 721 whose output is sent to mixer 703 which mixes the low noise amplified signal output from the LNA 721 with the RF carrier signal from the buffer 705.

The output of the mixer 703 is passed to the high pass filter (HPF) 712 that filters out the DC component and low frequency components of the signal, and then the output of the HPF 712 is sent to the intermediate frequency amplifier (IF amplifier) 713 which is configured to amplify the received signal. The amplified output signal from the IF amplifier 713 is provided to the low pass filter (LPF) 722 of the ASK receiver 720, and the output low pass filtered signal from LPF 722 is provided to another intermediate frequency amplifier 723 of the ASK receiver 720 which is configured to amplify the low pass filtered signal output from the LPF 722. As shown in FIG. 7, the IF amplifier 723 of the ASK receiver 720 is provided between the LPF 722 and the ASK demodulator 724.

Referring yet still to FIG. 7, the gain controller signal from IF amplifier 723 of the ASK receiver 720 controls the low noise amplifier (LNA) 721 that receives the filtered backscatter signal. The gain controller signal in one embodiment switches between high gain and low gain state of the LNA 721. For example, ifIF amplifier 723 has high gain, then the gain controller signal to the LNA 721 switches the LNA 721 to low gain operation, and vice versa. As discussed above, the output of the IF amplifier 723 of the ASK receiver 720 is provided to the ASK demodulator 724 of the ASK receiver 720 which is configured to demodulate (or recover the data) the output signal from the IF amplifier 723.

That is, as shown in FIG. 7, the RX enable line to the ASK receiver 720 is configured to tum on after the TX enable line where the tum on signal from the control unit 410 (FIG. 4) is received in the reader device/receiver unit such that with the receive enable signal from the control unit 410, the data out line (i.e., the output of the ASK demodulator 724) of the ASK receiver 720 provides the data or signal associated with the monitored glucose level based on the raw current signals from the glucose sensor.

Referring back to the Figures and as described above, in one aspect, the on-body patch device may include a power supply to power the electronic components as well as the sensor, or alternatively, the on-body patch device may not includes a separate dedicated power supply and rather, include a self-powered sensor as described in further detail in U.S. Patent Application No. 12/393,921 filed February 27, 2009 (with Publication No. US 2010/0213057 A1). In certain aspects, for configurations of the on-body
patch device that includes a power supply, the on-body patch device may be configured to listen for the RF control command (ping signal) from the reader device. More specifically, an On/Off Key
(00K) detector may be provided in the on-body patch device which is turned on and powered by the battery to listen for the RF control command or the ping signal from the reader device. Additional details if the OOK detector are provided in U.S. Patent Publication No. 2008/0278333. In certain aspects, when the RF control command is
detected, on-body patch device determines what response packet is necessary, and generates the response packet for transmission back to the reader device. In this embodiment, the sensor is always turned on and configured to continuously receive power from the power supply or the battery of the on-body patch device.
However, the sampled current signal from the sensor may not be transmitted out to the reader device/receiver unit until the on-body patch device receives the RF power (from the reader device/receiver unit) to enable the transmission of the data to the reader device. In one embodiment, the battery may be a rechargeable battery configured to be charged when the on-body patch device received the RF power (from the reader device/receiver unit).

In certain embodiments, the on-body patch device does not include an RF communication chip, nor any other dedicated communication chip to allow for wireless transmission separate from being powered on based on the RF power received from the reader device/receiver unit and transmitting the backscatter response packet to the reader device.

Referring again to FIG. 7, in a further embodiment of the present disclosure, an RF transmitter chip or an ASK transmitter may be provided to the reader device/receiver unit 220 (FIG. 2) to replace the SAW resonator 701, the oscillator 702, the mixer 703, the LC power splitter 704, the buffer 705, the power amplifier 706, the high pass filter (HPF) 712, and the IF amplifier 713 shown in FIG. 7. More specifically, in this embodiment of the reader device, the RF transmitter chip may be coupled to a crystal which provides the frequency reference base for generating the RF carrier signal to receive the backscatter from the on-body patch device, and also to send the control commands (ping signals) to the on-body patch device.

In the embodiment discussed above, in aspects of the present disclosure, the RF transmitter chip or unit may be coupled to the LC power splitter, a balun and the loop antenna similar to the LC power splitter 707, the balun 709, and the loop antenna 708 shown in FIG. 7, in addition to a SAW filter and ASK receiver similar to the SAW filter 711 and ASK receiver 720 shown in FIG. 7. However, in contrast to the configuration shown in FIG. 7, in the alternate embodiment, another crystal may be coupled to the ASK receiver to provide the frequency reference base for receiving the backscatter signal from the on-body patch device.

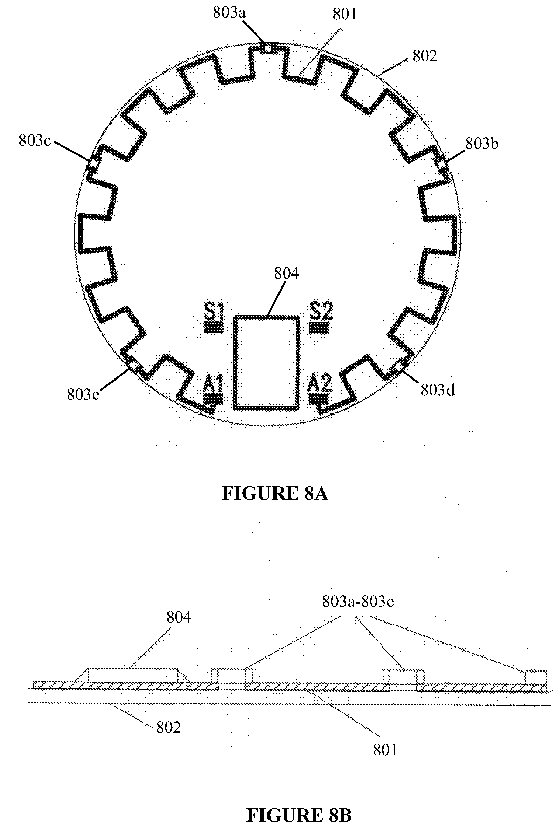

FIGS. 8A and 8B illustrate a top view and a side view, respectively, of antenna and electronic circuit layout of the on-body patch device including an sensor and sensor electronics assembly for use in the analyte monitoring systems of FIGS. 1 and 2 in one aspect of the present disclosure. Referring to FIGS. 8A and 8B, the loop antenna and circuit layout of the on-body patch device in one embodiment includes a conductive layer 801, such as a PCB copper trace, provided on a substrate 802, and further includes, a plurality of inductors 803a-803e disposed on the substrate and electrically connected to the conductive layer 801 in a loop configuration. In one aspect, the inductors 803a-803e are spaced equidistantly from each other around the loop configuration. In a further aspect, the inductors 803a-803e may not be equidistantly spaced apart from each other in the loop configuration. Also shown in FIGS. 8A and 8B is a data processor or controller 804 in electrical communication with the conductive layer 801 for processing signals from the sensor (not shown) and interfacing with the sensor in addition to processing the control commands from the reader device/receiver unit and generating and/or transmitting the backscatter response data packet to the reader device.

Accordingly, in aspects of the present disclosure, loop antenna configurations are provided for a passive glucose sensor and a low power glucose reader device/receiver unit at Ultra High Frequency (UHF) frequency bands, providing an on-demand glucose data acquisition system that includes the reader device/receiver unit which is configured to generate a strong near electromagnetic field to power the passive glucose sensor, and further provide a weak far electromagnetic field such that the strength of the generated magnetic field at a far distance, such as approximately 3 meters away from the on-body patch device, including the sensor is in compliance with the regulated radiation level.

In certain embodiments, the on-body patch device antenna may be printed as an internal conductive layer of a printed circuit board surrounded by the ground plane on the top and bottom layers. That is, in one aspect, the top and bottom conductive layers may be separated by layers of dielectrics and a conductive layer of loop antenna disposed therebetween. Further, the antenna for the on-body patch device may be printed on the top conductive layer of the printed circuit in series with a plurality of inductors chips, such as, for example, but not limited to, five inductor elements.

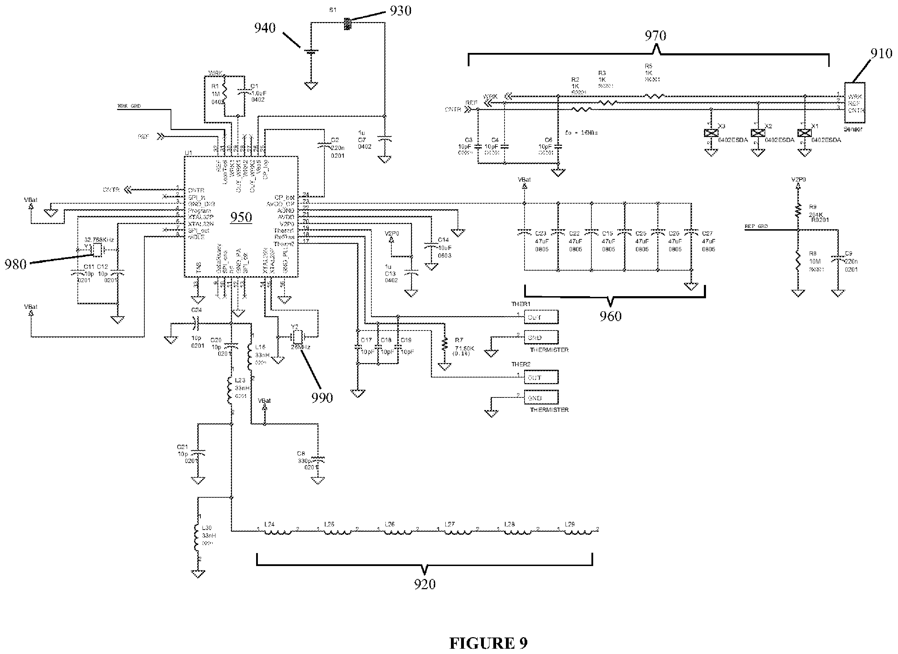

FIG. 9 illustrates an exemplary circuit schematic of the on-body patch device including an sensor and sensor electronics assembly in accordance with aspects of the present disclosure. Referring to the Figure, in one embodiment the sensor contacts 910 are provided to establish contact with the various electrodes of the sensor including working electrode, reference electrode and counter electrode. Also shown is an RF transmission antenna 920 operatively coupled to the control unit 950. In certain embodiments, the control unit 950 may be implemented as application specific integrated circuits (ASICs), or include microprocessors or both. An activation switch 930, described in further detail below, is also shown in FIG. 9 along the electrical path from the power supply 940 for switching on or turning on the sensor electronics of the on-body patch device.

Referring still to FIG. 9, also shown in analog front end circuitry/components 970 coupled to the sensor contacts 910 for processing the raw current signals generated by the analyte sensor and detected at the sensor contacts 910. Additional passive storage capacitors 960 coupled to the power supply such as a battery is shown. In addition, crystal oscillators 980, 990 are provided as shown in FIG. 9, where in certain embodiments, crystal oscillator 980 is configured to provide clock signals for the state machine in the ASIC 950, while crystal oscillator 990 may be configured to provide frequency reference for the RF communication components within the ASIC 950.

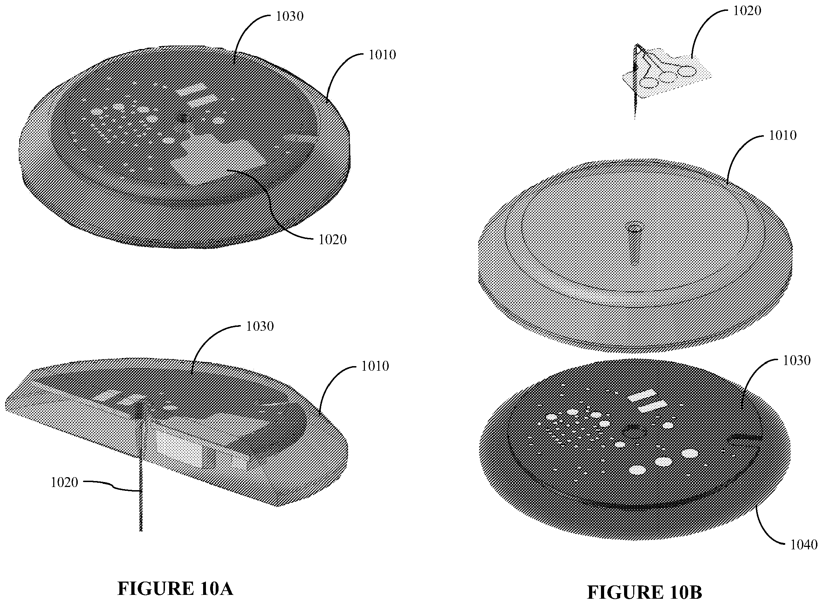

FIG. 10A is a perspective view of the components of the an on-body patch device including sensor and sensor electronics assembly in accordance with one aspect of the present disclosure. Referring to FIG. 10A, an integrated sensor and sensor electronics assembly/on-body patch device 110 of FIG. 1 in one embodiment is shown. As can be seen, the housing 1010 in one embodiment is substantially shaped such that the height profile is minimized (for example, to less than or equal to approximately 10 mm, e.g., about 4 mm or less). For example, as shown in the figures, the housing of the integrated assembly may have a dome-like shape, or otherwise tapered shape. A height dimension may be at most about 4 mm, and may taper (gradually or step wise) to heights less than about 4 mm, e.g., 3mm or less, e.g., 2 mm or less, e.g., 1 mm or less.

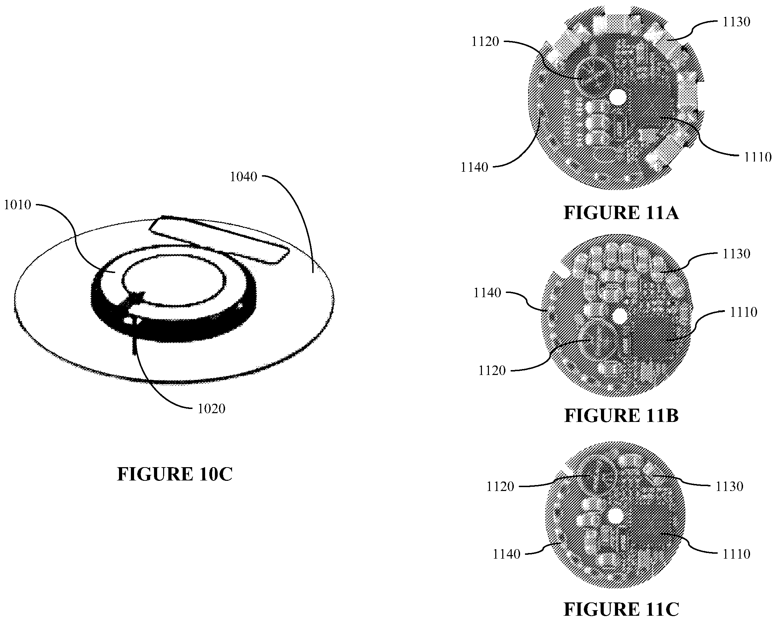

Referring back to FIG. 10A, in one embodiment, the analyte sensor 1020 is assembled (e.g., provided to the user) with the sensor electronics 1030 and provided within the housing 1010. Furthermore an adhesive (single sided or two sided) layer 1040 (FIG. 10C) may be provided on a lower surface of the housing 1010 to provide secure positioning of the housing 1010 on the skin surface during and after sensor deployment. As discussed in further detail below, the integrated sensor and sensor electronics assembly/on-body patch device 110 may be positioned (e.g., during manufacture to provide to the user) within the housing of an insertion device, avoiding the need for a user to align, position, or otherwise connect or couple the sensor and sensor electronics to the insertion device prior to the insertion of the sensor and turning on the sensor electronics. Accordingly, potential misuse, misalignment of the sensor relative to the introducer of the insertion device, or errors and difficulties in use of the integrated assembly by the user may be avoided.

FIG. 10B is another perspective view of the components of the on-body patch device including sensor and sensor electronics assembly in accordance with one aspect of the present disclosure. As shown in the Figure, each component of the integrated assembly is separated to illustrate the relative position of each component, in one embodiment. As discussed in further detail below, it can be seen in one embodiment that the sensor 1020 includes a bent configuration, whereby at least a portion of the body of the sensor is maintained in a direction substantially planar to the surface of the skin. In one aspect, this configuration allows for the low profile dimension of the housing 1010 that includes the sensor 1020 such that the protrusion of the housing 1010, when positioned on the skin surface of the user, is minimized. Accordingly, the sensor 1020 may be bent, or may be bendable, from about 1 degree to about 90 degrees or more.

FIG. 10C is another perspective view of the assembled on-body patch device including sensor and sensor electronics assembly in accordance with one aspect of the present disclosure. As shown in FIG. 10C, after positioning the integrated sensor and sensor electronics assembly, the adhesive layer 1040 may be configured to substantially fixedly retain the integrated assembly 110 on the skin surface such that movement of the sensor 1020 during the course of wearing the device is minimized. In one aspect, the adhesive layer 1040 may be configured to provide a substantially water tight seal between the integrated assembly 110 and the skin surface during the predetermined time period of wear such that the likelihood of the integrated assembly 110 detaching from the skin surface is minimized.

FIGS. 11A-11C illustrate circuit layouts for the sensor electronics assembly in the on-body patch device including sensor and sensor electronics assembly in accordance with embodiments of the present disclosure. Referring to FIGS. 11A-11C, embodiments of the sensor electronics of the integrated assembly includes dimensions that are optimized for reduction and thus maximized for comfort in use and wear. For example, embodiments of the sensor electronics shown in FIGS. 11A-11C may include a diameter of approximately 25mm or less (typical size of a quarter coin, for example), e.g., 20 mm or less, or 15 mm or less. As shown, for example, the control unit including an application specific integrated circuit (ASIC) 1110 is provided in electrical contact with a plurality of RF communication transmission capacitors 1130 positioned, for example, substantially around the outer periphery of the flexible circuit board. Depending upon the size of the circuit board and/or RF transmission requirement, RF transmission capacitors 1130 of different capacitance may be provided. For example, FIG. 11A illustrates RF transmission capacitors 1130 of 600µF, while the FIGS. 11B and 11C illustrate RF transmission capacitors 1130 having approximately 610µF and 240µF, respectively.

Referring back to the Figures, also shown is a battery 1120 configured to provide the necessary power for the operation of the sensor electronics, and may include a single use coin-cell type battery that is disposable after single use, but which is sufficient to provide the necessary power to operate the integrated sensor and sensor electronics assembly 110 (FIG. 1) during the desired time period (for example, such as 5 days or 7 days or longer). Additionally, further shown in FIGS. 11A-11C are RF antennas 1140 that are positioned, in one embodiment substantially around the circumference of a portion of the flexible circuit board.

Accordingly, in aspects of the present disclosure, the circuit layout of the sensor electronics may be optimized to minimize the surface area of the circuit board (and thus the overall size of the integrated assembly), by positioning the various components in the manner as shown in FIGS. 11A-11C.

FIGS. 12A-12B illustrate pre-deployment and post insertion configurations of the insertion device for positioning the on-body patch device including sensor and sensor electronics assembly in accordance with embodiments of the present disclosure. Referring to FIG. 12A, insertion device 1200 in one embodiment includes a housing or body 1210 and a cap 1220 which is configured to provide closure or seal on the open end of the insertion device. As shown, the insertion device 1200 may be configured for sensor insertion and sensor electronics assembly positioning in a direction substantially perpendicular to the skin surface.

Referring to FIG. 12B, when a force, e.g., a manual force, is applied upon the top end of the housing 1210 in the direction as shown by arrow 1240, and with the open end of the housing on the skin surface 1230, the integrated sensor and sensor electronics assembly provided within the housing (not shown) is configured to come into contact with the skin surface 1230. Furthermore, the force applied as discussed above also may be configured to move the introducer (not shown) within the housing in the same direction as shown by arrow 1240 to pierce the skin surface 1230 and position the sensor in fluid contact with an analyte of the user.

Further details of the mechanism associated with the insertion device for sensor insertion and sensor electronics assembly positioning is shown and described below in conjunction with FIGS. 12C-12G which illustrate cross sectional perspective views of the operation of the insertion device for deploying the on-body integrated sensor and sensor electronics assembly in accordance with embodiments of the present disclosure.

As shown in these figures, in response to the force applied on the insertion device housing 1210, the introducer 1260 is driven in a direction substantially perpendicular to the skin surface 1230, and along with the movement of the introducer 1260, the sensor 1280 and the sensor electronics assembly 1270 are moved in the same direction. When the bottom surface of the sensor electronics assembly 1270 comes into contact with the skin surface 1230, the bottom surface is maintained in an adhered relationship with the skin surface 1230 by, for example, the adhesive layer 1290 (FIG. 12G). Moreover, also shown in the Figures is a bias spring 1250 which, in one embodiment, is configured to retract the introducer needle from the insertion position to a retracted position which is an opposite direction from the direction indicated by arrow 1240 (FIG. 12B).

Referring back to the Figure, it can be seen that the introducer needle 1260 is substantially and entirely retained within the insertion device housing 1210 after sensor insertion, and thereafter, when the insertion device 1200 is removed from the skin surface 1230, the sensor electronics assembly 1270 is retained on the skin surface 1230, while the position of the sensor 1280 is maintained in fluid contact with the analyte of the user under the skin layer 1230.

Prior to activation of the integrated sensor and sensor electronics assembly for use, there may be a period of time from the manufacturing that the assembly may be in sleep or idle mode. With a power supply such as a battery integrated within the assembly, for reasons including cost optimization and prolonging shelf life, embodiments of the present disclosure include systems that are activated merely by positioning the sensor and electronics unit on a skin surface as described above, i.e., no additional action need be required of the user other than applying a force to housing 1210. As such, insertion of the sensor causes activation of the electronics unit. In certain embodiments, activation switch configurations are included which may be configured to be triggered, for example, by the insertion device activation, thereby turning on the integrated sensor and sensor electronics assembly into an active mode.

For example, FIGS. 13A-13B illustrate embodiments of a power supply switch mechanism including conductive plugs of the on-body patch device including sensor and sensor electronics assembly in accordance with embodiments of the present disclosure. As shown, the sensor electronics assembly circuit board 1310 may be provided with a physical gap 1350 that breaks the electrical circuit between the power supply (e.g., battery) and the other circuitry of the sensor electronics assembly.

In one embodiment, when the predetermined force is applied on the insertion device as discussed above, a conductive portion 1320 provided within the housing of the sensor electronics may be moved in a direction as shown by arrow 1330 such that electrical contact is established in the physical gap 1350 on the circuit board, by for example, the conductive portion 1320 coming into physical contact with the conductive portions 1360 of the circuit board. In this manner, in one embodiment, the electrical path from the power supply and the remaining circuitry on the circuit board of the sensor electronics is completed, thereby powering the sensor electronics.

By way of another example, referring to FIG. 13B, the conductive portions 1360 of the circuit board are provided on the board itself, and the conductive plug 1340, for example, when pushed into the cavity 1350, establishes electrical contact between the conductive portions 1360 of the circuit board.

In one embodiment, as discussed above, the actuation of the insertion device to position the sensor and sensor electronics assembly triggers the switch mechanism shown in FIGS. 13A and 13B by also moving the conductive portion 1320 or the conductive plug 1360 in the direction complimentary to the direction of the introducer movement, and thereby switching on the sensor electronics. Within the scope of the present disclosure, the activation of the sensor electronics by moving the conductive portion 1320 or the conductive plug may include a separate procedure, where after positioning the sensor and the sensor electronics assembly on the skin surface, a predetermined force is applied on the housing of the integrated sensor and sensor electronics assembly such that the desired movement of the conductive portion 1320 or the conductive plug 1360 may be achieved.

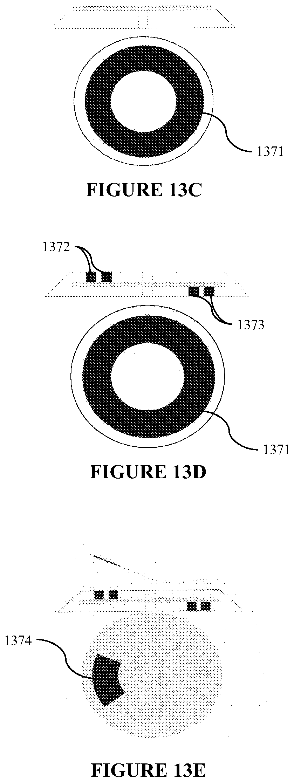

FIGS. 13C-13E illustrate another configuration of the power supply switch mechanism including conductive pads of the on-body patch device including sensor and sensor electronics assembly in accordance with embodiments of the present disclosure. Referring to FIG. 13C, an exposed conductive ring 1371 may be provided and configured to contact the surface of the circuit board in the sensor electronics such that, the insertion device activation positions the conductive ring 1371 on the surface of the circuit board so as to complete the electrical contact of the sensor electronics assembly (by for example, manual force applied on the insertion device placing the conductive ring in contact with the circuit board of the sensor electronics).

Referring to FIG. 13D, in another aspect, electrical contact pads 1372, 1373 may be provided to the circuit board in the sensor electronics assembly such that the mating of the contact pads with the conductive ring 1371 switches on the sensor electronics device to provide power to the device from its power source. FIG. 13E shows yet another configuration of the switch activation mechanism in accordance with the present disclosure, where a portion of the conductive ring 1374 is selectively positioned and provided to establish electrical contact in the device.

As discussed, each of the activation configuration described above includes a break in the circuitry from the power source such that the power supply is not drained when the device is not in use, and upon activation, the break in the electrical contact is completed, thereby powering the device and activating it for operation.

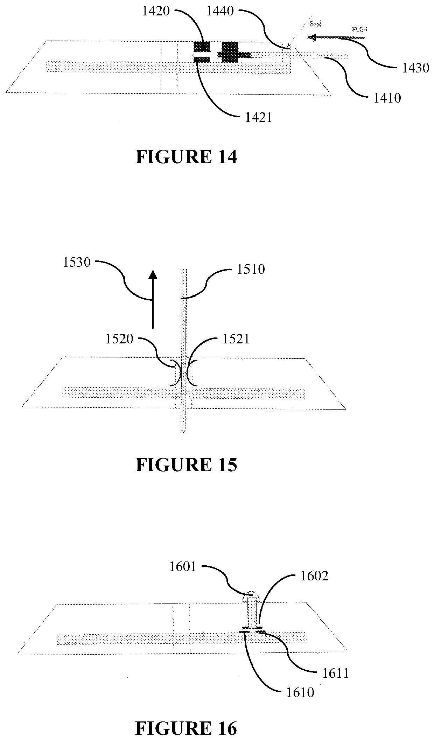

FIG. 14 illustrates a power supply switch mechanism including an internal switch with a push rod activation of the on-body patch device including sensor and sensor electronics assembly in accordance with embodiments of the present disclosure. As shown, in one embodiment, push rod 1410 may be provided and positioned in the sensor electronics such that when a force is applied in the direction as shown by arrow 1430, the push rod 1410 is displaced in the same direction, and completes the electrical contact between the two contacts 1420, 1421. In one aspect, the push rod 1410 may be provided within a seal 1440 such as an O-ring or similar components.

FIG. 15 illustrates a power supply switch mechanism including introducer retraction trigger activation of the on-body integrated sensor and sensor electronics assembly in accordance with embodiments of the present disclosure. As shown, a nonconducting needle or device 1510 is provided to physically separate two electrical contacts 1520, 1521. Each of the electrical contacts 1520, 1521 is biased or spring loaded to be urged towards each other, physically separated by the nonconducting needle 1510. Accordingly, when the nonconducting needle 1510 is retracted or pulled away from the sensor electronics assembly in the direction as shown by arrow 1530, the two electrical contacts 1520, 1521 are configured to contact each other, thereby completing the break in the circuit and establishing electrical connection to activate the sensor electronics assembly.

In one aspect, the nonconducting device or needle 1510 may include, for example, but not limited to, glass, plastic or any other material suitable to separate two electrical contacts and provide insulation therebetween.

FIG. 16 illustrates a power supply switch mechanism with a contact switch of the on-body patch device including sensor and sensor electronics assembly in accordance with embodiments of the present disclosure. As shown, in a further aspect, there is provided an electronic switch 1601 (that is configured to draw an insubstantial amount of power from the sensor electronics power supply), and when triggered, completes the break between the contacts 1610, 1611 by physically contacting the two contacts 1610, 1611 with the activation component 1602 that completes the circuit in the sensor electronics from its power supply such as battery to activate the device for operation.

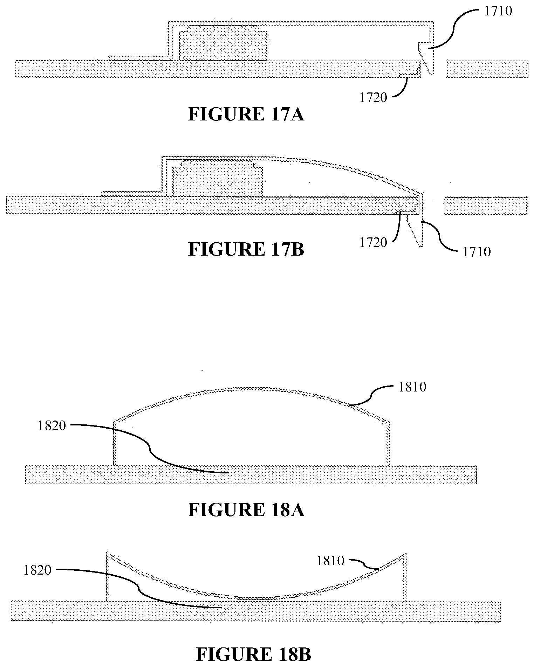

FIGS. 17A-17B illustrate a power supply switch mechanism with a battery contact locking mechanism of the on-body patch device including sensor and sensor electronics assembly in accordance with embodiments of the present disclosure. Referring to the Figures, in still another aspect, the battery contact of the sensor electronics may be provided with a barbed tab 1710. In post manufacturing shelf mode when the device is nonoperational, the tab 1710 is positioned within the sensor electronics housing in the position as shown in FIG. 17A so that it is not in contact with the conductive contact 1720 of the sensor electronics circuit board. When in use as shown in FIG. 17B, the tab 1710 may be biased such that it physically contacts the conductive contact 1720 on the circuit board, thereby closing the circuit to/from the battery/power source and thus activating or switching on the sensor electronics. As shown in the Figures, the tab 1710 may be configured that upon biasing to establish contact with the conductive contact 1720, it locks or latches with the conductive contact 1620 and the circuit board so as to maintain the electrical connection.

FIGS. 18A-18B illustrate a power supply switch mechanism with a bi-modal dome switch of the on-body patch device including sensor and sensor electronics assembly in accordance with embodiments of the present disclosure. Yet in another embodiment, a bi-modal dome shaped switch 1810 is provided on the circuit board of the sensor electronics assembly such that, when pressed down (as shown in FIG. 18B), the dome shaped layer 1810 (which may include, for example, a thin sheet metal dome) may be configured to retain the concave shape as shown in FIG. 18B and effectively closing the circuit on the circuit board at the contact point 1820. In one aspect, the dome shaped layer 1810 may be configured to shunt to short two or more electrical contacts at the contact point 1820 of the circuit board. Alternatively, the dome shaped layer 1810 may be connected to the circuit board such that one end of the dome shaped layer 1810 is in contact with one of the two or more open electrical contacts, and the depression of the dome shaped layer 1810 closes the circuit on the circuit board by physically contacting the other one or more of the open electrical contacts.

In the manner described above, in accordance with various embodiments of the present disclosure, sensor electronics activation switch configurations are provided that may be triggered or activated automatically or semi-automatically in response to the activation of the insertion device described above, or alternatively, may be separately activated by the user by, for example, depressing upon a portion of the housing or switch provided on the housing of the sensor electronics. Accordingly, power consumption may be optimized for the sensor electronics assembly while improving post manufacturing shelf life of the device prior to use or activation.

As described above, in certain aspects of the present disclosure, discrete glucose measurement data may be acquired on-demand or upon request from the reader device, where the glucose measurement is obtained from an *in vivo* glucose sensor transcutaneously positioned under the skin layer of a patient or a subject, and further having a portion of the sensor maintained in fluid contact with the interstitial fluid under the skin layer. Accordingly, in aspects of the present disclosure, the patient or the user of the analyte monitoring system may conveniently determine real time glucose information at any time, using the RFID communication protocol as described above.

In the manner described above, in accordance with various embodiments of the present disclosure, discrete glucose measurements may be obtained within the need for lancing or performing fingerprick test for access to blood sample each time a measurement is desired. The analyte monitoring system described in further aspects may be configured to log or store glucose data monitored by the analyte sensor continuously over a predetermined or programmable time period, or over the life of the sensor without user intervention, and which data may be retrieved at a later time as desired. Furthermore, output indications such as audible, visual or vibratory alerts may be provided to inform the user of a predetermined condition or when the monitored glucose level deviates from a predefined acceptable range (for example, as warning indication of low glucose or high glucose level).

The various processes described above including the processes operating in the software application execution environment in the analyte monitoring system including the on-body patch device, sensor electronics, the reader device, the receiver unit, data processing module and/or the remote terminal performing one or more routines described above may be embodied as computer programs developed using an object oriented language that allows the modeling of complex systems with modular objects to create abstractions that are representative of real world, physical objects and their interrelationships. The software required to carry out the inventive process, which may be stored in a memory or storage device of the storage unit of the various components of the analyte monitoring system described above in conjunction to the Figures including the on-body patch device, the reader device, the data processing module, various described communication devices, or the remote terminal may be developed by a person of ordinary skill in the art and may include one or more computer program products.

In still another aspect, the methods, devices and systems described above may be configured to log and store (for example, with an appropriate time stamp and other relevant information such as, for example, contemporaneous temperature reading)) the real time analyte data received from the analyte sensor, and may be configured to provide the real time analyte data on-demand by using, for example a device such as a blood glucose meter or a controller discussed above that is configured for communication with the on-body integrated sensor and sensor electronics assembly.

That is, in one embodiment, real time data associated with the analyte being monitored is continuously or intermittently measured and stored in the integrated on-body sensor and sensor electronics assembly, and upon request from another device such as the receiver unit or the reader device/receiver unit (operated by the user, for example) or any other communication enabled device such as a cellular telephone, a personal digital assistant, an iPhone, a Blackberry device, a Palm device such as Palm Treo, Pro, Pre, Centro), or any other suitable communication enabled device which may be used to receive the desired analyte data from the on-body integrated sensor and sensor electronics assembly while being worn and used by the user. In one aspect, such communication enabled device may be positioned within a predetermined proximity to the integrated on-body sensor and sensor electronics assembly, and when the communication enabled device is positioned within the predetermined proximity, the data from the integrated on-body sensor and sensor electronics assembly may be transmitted to the communication enabled device. In one aspect, such data communication may include inductive coupling using, for example, electromagnetic fields, Zigbee protocol based communication, or any other suitable proximity based communication techniques. In this manner, glucose on-demand mode may be provided such that the information associated with contemporaneously monitored analyte level information is provided to the user on-demand from the user.

In this manner, in embodiments of the present disclosure, the size and dimension of the on-body sensor electronics may be optimized for reduction by, for example, flexible or rigid potted or low pressure/low temperature overmolded circuitry that uses passive and active surface mount devices for securely positioning and adhering to the skin surface of the user. When flexible circuitry is with or in the overmold, the sensor electronics may includes the analyte sensor and/or other physiological condition detection sensor on the flex circuit. Furthermore in embodiments of the present disclosure, one or more printed RF antenna may be provided within the sensor electronics circuitry for RF communication with one or more remote devices, and further, the device operation and/or functionalities may be programmed or controlled using one or more a microprocessors, or application specific integrated circuits (ASIC) to reduce the number of internal components.

Embodiments of the present disclosure include one or more low pressure molding materials that directly encapsulate the integrated circuits or the sensor electronic components. The thermal process entailed in the encapsulation using the low pressure molding materials may be configured to shield temperature sensitive components such as, for example, the analyte sensor or other components of the sensor electronics from the heat generated during the thermal overmolding process. Other techniques such as injection molding and/or potting may be used.

In another aspect, the sensor electronics may be molded using optical techniques such as with a UV cured material, for example, or using two photon absorption materials, which may also be used to reduce the dead or unused volume surrounding the sensor electronics within the housing of the device such that the reduction of its size and dimension may be achieved. Moreover, the sensor electronics may be configured to reduce the number of components used, for example, by the inclusion of an application specific integrated circuit (ASIC) that may be configured to perform the one or more functions of discrete components such as a potentiostat, data processing/storage, thermocouple/thermister, RF communication data packet generator, and the like. Additionally, a field programmable gate array (FPGA) or any other suitable devices may be used in addition to the ASIC in the sensor electronics to reduce the on-body device dimension.

Also, embodiments of the present disclosure includes analyte sensors that may be fabricated from flex circuits and integrated with the sensor electronics within the device housing, as a single integrated device. Example of flex circuits may include evaporated or sputtered gold on polyester layer, single or multi-layer copper or gold on polymide flex circuit. When the sensor fabricated from a copper or gold polymide flex circuit, gold or other inert material may be selectively plated on the implantable portion of the circuit to minimize the corrosion of the copper. In aspects of the present disclosure, the flex circuit may be die or laser cut, or alternatively chemically milled to define the sensor from the flex circuit roll.

A further configuration of embodiments of the present disclosure includes RF communication module provided on the flex circuit instead of as a separate component in the sensor electronics. For example, the RF antenna may be provided directly on the flex circuit by, such as surrounding the sensor electronics components within the housing on the flex circuit, or folded over the components, and encapsulated with the electronic components within the housing of the device.

In accordance with embodiments of the present disclosure, the integrated sensor and sensor electronics assembly may be positioned on the skin surface of the user using an insertion device. For example, automated or semi-automated, spring biased and/or manual insertion device may be provided to deploy the sensor and the sensor electronics such that the implantable portion of the sensor is positioned in fluid contact with the analyte of the user such as the interstitial fluid, while the housing of the sensor electronics is securely positioned and adhered to the skin surface. In embodiments of the present disclosure, the sensor electronics device (for example, a transmitter unit of an analyte monitoring system) may be switched to an operational state or condition (from an inactive, shelf mode) upon deployment of the integrated assembly by the insertion device.

In one aspect, integrated sensor and sensor electronics assembly may be preloaded or otherwise pre-assembled within the insertion device, such that, when in use, the user may, by a single operation of the insertion device, deploy the integrated sensor and sensor electronics assembly, without the need to couple the integrated assembly with the insertion device prior to deployment.

In one aspect, the integrated sensor and sensor electronics assembly and the insertion device may be sterilized and packaged as one single device and provided to the user. Furthermore, during manufacturing, the insertion device assembly may be terminal packaged providing cost savings and avoiding the use of, for example, costly thermoformed tray or foil seal. In addition, the inserter device may include an end cap that is rotatably coupled to the insertion device body, and which provides a safe and sterile environment (and avoid the use of desiccants for the sensor) for the sensor provided within the insertion device along with the integrated assembly. Also, the insertion device sealed with the end cap may be configured to retain the sensor within the housing from significant movement during shipping such that the sensor position relative to the integrated assembly and the insertion device is maintained from manufacturing, assembly and shipping, until the device is ready for use by the user.

Moreover, as discussed above, the insertion device in embodiments of the present disclosure includes a sharp needle or introducer for aiding the transcutaneous insertion of the sensor through the skin layer of the user. The sharp needle or the introducer may be configured to be retracted within the insertion device housing after deployment to permit movement, such as tilting or angled movement, to position the adhesive on the housing of the sensor electronics onto the skin surface of the user without the introducer interfering such movement. Also, by retaining the introducer within the insertion device housing after insertion, the disposal of the used introducer may be safer, without presenting possible biohazard concerns.

Also, in embodiments of the present disclosure the sharp needle or the introducer is not visible to the user prior to, during and after the use of the insertion device to position the sensor and the sensor electronics. As such, potential for perceived pain associated with when the sharp needle is visible is minimized.

In a further embodiment, the insertion device may be configured for manual deployment with spring biased or automatic retraction of the introducer. That is, sensor insertion, the user may apply a predetermined amount of pressure upon the housing of the insertion device to insert the introducer and the sensor, the applied pressure sufficient to pierce through the skin layer of the user, and the device housing configured such that the applied pressure or the distance traveled by the introducer is predetermined (for example, by the use of a stopper or a protrusion within the inner wall of the insertion device that effectively stops of blocks further downward movement of the introducer towards the skin piercing direction after the introducer has reached a predetermined distance. In one aspect, the applied pressure may be configured to also press down upon a spring or a bias mechanism provided within the housing of the insertion device such that, when the applied pressure is released, the introducer is automatically retracted to its original predeployment position within the housing of the insertion device, by the return force from the spring or bias mechanism.

In this manner, consistent and repeatable insertion depth for the placement of the analyte sensor may be achieved. Furthermore, the insertion device housing (for example, a plastic or a combination of plastic and metal housing) may not be under the stress of spring tension since the bias spring provided for retraction of the introducer is, in the predeployment state, unbiased and in a relaxed state.

In a further embodiment, two sided adhesive layer may be provided along the other periphery of the insertion device that is positioned to be in contact with the skin surface of the user such that, proper alignment and positioning of the introducer, the sensor and the sensor electronics assembly may be provided before and during the sensor positioning process, in addition to increased comfort and breathability of the material once adhered to the skin layer of the user.

In one embodiment, an integrated analyte monitoring device assembly may comprise an analyte sensor for transcutaneous positioning through a skin layer and maintained in fluid contact with an interstitial fluid under the skin layer during a predetermined time period, the analyte sensor having a proximal portion and a distal portion, and sensor electronics coupled to the analyte sensor, the sensor electronics comprising a circuit board having a conductive layer and a sensor antenna disposed on the conductive layer, one or more electrical contacts provided on the circuit board and coupled with the proximal portion of the analyte sensor to maintain continuous electrical communication, and a data processing component provided on the circuit board and in signal communication with the analyte sensor, the data processing component configured to execute one or more routines for processing signals received from the analyte sensor, the data processing component configured to control the transmission of data associated with the processed signals received from the analyte sensor to a remote location using the sensor antenna in response to a request signal received from the remote location.

The proximal portion of the analyte sensor and the circuit board may be encapsulated.

The proximal portion of the analyte sensor and the circuit board may be encapsulated with a potting material.

The circuit board may include an upper layer and a lower layer, where the conductive layer is disposed between the upper layer and the lower layer.

The antenna may include a loop antenna or a dipole antenna.

The antenna may be printed on the conductive layer.

In one aspect, the assembly may include a plurality of inductive components coupled to the sensor antenna on the conductive layer of the circuit board.

The plurality of inductive components may be coupled in series to the sensor antenna.

The plurality of inductive components may be positioned substantially around an outer edge of the circuit board.

The circuit board may be substantially circular, and the plurality of components may be positioned around the outer circumference of the circular circuit board.

Each of the plurality of the inductive components may be positioned substantially equidistant to each other on the circuit board.

Moreover, the assembly may include a power supply to provide power to the sensor electronics.

The data processing component may include an application specific integrated circuit (ASIC) disposed on the circuit board and configured to process signals from the analyte sensor.

The data processing component may include a state machine.

The state machine may be configured to execute one or more programmed or programmable logic for processing the signals received from the analyte sensor.

The analyte sensor may include a glucose sensor.

In another embodiment, an analyte data acquisition device may comprise a control unit configured to generate a control command based on a carrier signal, an antenna section coupled to the control unit to transmit the control command with the carrier signal and to receive a backscatter response data packet using the carrier signal, and a receiver section coupled to the antenna section and the control unit to process the received backscatter response data packet and to generate an output glucose data.

The control unit may include a signal resonator coupled to an oscillator, and configured to generate RF power.

The signal resonator may include a surface acoustic wave resonator.

The generated RF power and the control command may be transmitted with the carrier signal.

The control command may include an RF control command transmitted with the carrier signal to a remote location.

The backscatter response data packet may be received from the remote location when the antenna is positioned no more than approximately 25.4cm (ten inches) from the remote location.

The antenna may be positioned about 12.7cm (five inches) or less from the remote location.

The antenna section may include one or more of a loop antenna, or a dipole antenna.

The control unit may be configured to generate the carrier signal.

The receiver section may include a filter to filter the received backscatter response data packet.

A further aspect may include an output unit operatively coupled to the control unit to output an indication corresponding to the generated glucose data.

The outputted indication may include one or more of a visual output, an audible output, a vibratory output, or one or more combinations thereof.

The control unit may generate a receipt confirmation signal upon successful receipt of the backscatter response data packet.

The generated receipt confirmation signal may be output to the user.

In another aspect, the device may further include a storage device coupled to the control unit to store the generated control command, carrier signal, the received backscatter response data packet, the generated output glucose data, or one or more combinations thereof.

The storage device may include a nonvolatile memory device.

The control unit may include a microprocessor.

The control unit may include an application specific integrated circuit.

Yet another aspect may include a strip port for receiving an *in vitro* blood glucose test strip, the strip port including an electrical connection in signal communication with the control unit.

The control unit may be configured to process a sample on the test strip to determine a corresponding blood glucose level.

In another embodiment, an integrated analyte monitoring device may comprise a sensor electronics assembly including an analyte sensor, a power supply, an activation switch operatively coupled to the power supply and the analyte sensor, a controller unit in electrical contact with the analyte sensor and the activation switch having one or more programming instructions stored therein for execution, the controller unit configured to process one or more signals received from the analyte sensor when the activation switch is triggered, and an insertion device including a housing, an introducer coupled to the housing configured to move between a first position and a second position, and a bias mechanism operatively coupled to the housing configured to automatically retract the introducer from the second position to the first position.

The sensor electronics assembly may be retained entirely within the housing of the insertion device prior to the introducer movement from the first position to the second position.

The activation switch may be not triggered until the introducer has reached the second position.

The analyte sensor may include a glucose sensor.

The activation switch may be triggered after the introducer has reached the second position, and prior to the introducer retraction from the second position to the first position.

The introducer may engage with the analyte sensor during its movement from the first position to the second position, and further, wherein the introducer disengages from the analyte sensor during its movement from the second position to the first position.

The movement of the introducer from the first position to the second position may be in response to a manual force applied on the housing.

The bias mechanism may include a spring.

A further aspect may include an adhesive layer provided on a bottom surface of the housing for placement on a skin layer.

The adhesive layer may be configured to retain the sensor electronics assembly on the skin layer for a predetermined time period.

The power supply may include a single use disposable battery.

The active operational life of the power supply may exceed the active operational life of the analyte sensor.

Moreover, one aspect may include a cap configured to mate with an open end of the housing of the insertion device.

When the cap is coupled to the housing, the interior space of the housing may be maintained in a substantially contaminant free environment.

The sensor electronics assembly may include a printed circuit board including a portion of the analyte sensor permanently connected thereto.

The controller unit may include an application specific integrated circuit (ASIC).

The movement of the introducer between the first position and the second position may be at an angle at approximately 90 degrees or less from a skin surface.

The sensor electronics assembly may include a housing having a height of less than approximately 4 mm.

Various other modifications and alterations in the structure and method of operation of this disclosure will be apparent to those skilled in the art without departing from the scope of the embodiments of the present disclosure.
Although the present disclosure has been described in connection with particular embodiments, it should be understood that the present disclosure as claimed should not be unduly limited to such particular embodiments. It is intended that the following claims define the scope of the present disclosure and that structures and methods within the scope of these claims be covered thereby.

## Claims

1. An integrated analyte monitoring device assembly (110, 211), comprising:
an analyte sensor (101, 250, 540, 1020) for transcutaneous positioning through a skin layer (210) and maintained in fluid contact with an interstitial fluid under the skin layer during a predetermined time period, the analyte sensor having a proximal portion and a distal portion; and
sensor electronics (102, 1030) coupled to the analyte sensor, the sensor electronics comprising:
a circuit board (1310) having a conductive layer (801) and a sensor antenna disposed on the conductive layer;
one or more electrical contacts (1360) provided on the circuit board and coupled with the proximal portion of the analyte sensor to maintain continuous electrical communication; and
a data processing component (610, 950) including an application specific integrated circuit (ASIC) provided on the circuit board and in signal communication with the analyte sensor, the data processing component configured to execute one or more routines for processing analyte related signals received from the analyte sensor, the data processing component configured to control the transmission of the data associated with the analyte related signals received from the analyte sensor to a remote location (220) using the sensor antenna only in response to a request signal received from the remote location; **characterised in that** the analyte sensor is coupled with the sensor electronics and provided to the user within a housing (1010) prior to positioning the analyte sensor through the skin layer.

2. The assembly (110, 211) of claim 1 wherein the proximal portion of the analyte sensor (101, 250, 540) and the circuit board (1310) are encapsulated.

3. The assembly (110, 211) of claim 2 wherein the proximal portion of the analyte sensor (101, 250, 540, 1020) and the circuit board (1310) are encapsulated with a potting material.

4. The assembly (110, 211) of claim 1 wherein the circuit board (1310) includes an upper layer and a lower layer, where the conductive layer (801) is disposed between the upper layer and the lower layer.

5. The assembly (110, 211) of claim 1 wherein the antenna includes a loop antenna or a dipole antenna.

6. The assembly (110, 211) of claim 1 including a power supply (1120) to provide power to the sensor electronics.

7. The assembly (110, 211) of claim 1 wherein the data processing component includes a state machine.

8. The assembly (110, 211) of claim 7 wherein the state machine is configured to execute one or more programmed or programmable logic for processing the signals data associated with the analyte related signals received from the analyte sensor (101, 250, 540, 1020).

9. The assembly (110, 211) of claim 1 wherein the data processing component (610, 950) is configured to transition from an inactive state to an active state upon detection of RF power from the remote location (220), and wherein the request signal for the data associated with the analyte related signals received from the remote location includes a radio frequency (RF) control signal.

10. The assembly (110, 211) of claim 9 wherein the RF power and the RF control signal are both received on a same carrier signal from the remote location (220).

11. The assembly (110, 211) of claim 10 wherein the sensor antenna is tuned such that when the data processing component (610, 950) transitions from the inactive state to the active state upon detection of the RF power from the remote location (220), the data associated with the analyte related signals is transmitted as a reflected backscatter signal to the remote location.

12. The assembly (110, 211) of claim 1 wherein the analyte sensor (101, 250, 540, 1020) includes a bent configuration, whereby at least the proximal portion of the body of the analyte sensor is maintained in a direction substantially planar to the surface of the skin.

## Patentansprüche

1. Integrierte Analytüberwachungsvorrichtungsbaugruppe (110, 211), die Folgendes umfasst:
einen Analytsensor (101, 250, 540, 1020) zur transkutanen Positionierung durch eine Hautschicht (210), der während einer vorbestimmten Zeitdauer in Fluidkontakt mit einer interstitiellen Flüssigkeit unter der Hautschicht gehalten wird, wobei der Analytsensor einen proximalen Abschnitt und einen distalen Abschnitt aufweist; und
Sensorelektronik (102, 1030), die mit dem Analytsensor gekoppelt ist, wobei die Sensorelektronik Folgendes umfasst:
eine Leiterplatte (1310) mit einer leitfähigen Schicht (801) und einer Sensorantenne, die auf der leitfähigen Schicht angeordnet ist;
einen oder mehrere elektrische Kontakte (1360), die auf der Leiterplatte vorgesehen und mit dem proximalen Teil des Analytsensors gekoppelt sind, um eine kontinuierliche elektrische Kommunikation aufrechtzuerhalten; und
eine Datenverarbeitungskomponente (610, 950) mit einer anwendungsspezifischen integrierten Schaltung (ASIC), die auf der Leiterplatte vorgesehen ist und in Signalverbindung mit dem Analytsensor steht, wobei die
Datenverarbeitungskomponente so konfiguriert ist, dass sie eine oder mehrere Routinen zum Verarbeiten von analytbezogenen Signalen, die von dem Analytsensor empfangen werden, ausführt; wobei die Datenverarbeitungskomponente so konfiguriert ist, dass sie die Übertragung der Daten, die den analytbezogenen Signalen zugeordnet sind, die von dem Analytsensor empfangen werden, zu einem entfernten Ort (220) unter Verwendung der Sensorantenne nur als Reaktion auf ein Anfragesignal steuert, das von dem entfernten Ort empfangen wird;
**dadurch gekennzeichnet, dass** der Analytsensor mit der Sensorelektronik gekoppelt ist und dem Benutzer in einem Gehäuse (1010) bereitgestellt wird, bevor der Analytsensor durch die Hautschicht angeordnet wird.

2. Baugruppe (110, 211) nach Anspruch 1, wobei der proximale Teil des Analytsensors (101, 250, 540) und die Leiterplatte (1310) verkapselt sind.

3. Baugruppe (110, 211) nach Anspruch 2, wobei der proximale Teil des Analytsensors (101, 250, 540, 1020) und die Leiterplatte (1310) mit einem Vergussmaterial verkapselt sind.

4. Baugruppe (110, 211) nach Anspruch 1, wobei die Leiterplatte (1310) eine obere Schicht und eine untere Schicht umfasst, wobei die leitfähige Schicht (801) zwischen der oberen Schicht und der unteren Schicht angeordnet ist.

5. Baugruppe (110, 211) nach Anspruch 1, wobei die Antenne eine Rahmenantenne oder eine Dipolantenne umfasst.

6. Baugruppe (110, 211) nach Anspruch 1, die eine Stromversorgung (1120) umfasst, um die Sensorelektronik mit Strom zu versorgen.

7. Baugruppe (110, 211) nach Anspruch 1, wobei die Datenverarbeitungskomponente eine Zustandsmaschine umfasst.

8. Baugruppe (110, 211) nach Anspruch 7, wobei die Zustandsmaschine dafür konfiguriert ist, eine oder mehrere programmierte oder programmierbare Logikeinheiten zum Verarbeiten der Signaldaten auszuführen, die den analytbezogenen Signalen zugeordnet sind, die von dem Analytsensor (101, 250, 540, 1020) empfangen werden.

9. Baugruppe (110, 211) nach Anspruch 1, wobei die Datenverarbeitungskomponente (610, 950) so konfiguriert ist, dass sie bei Erfassung von HF-Energie von dem entfernten Ort (220) aus einen inaktiven Zustand in einen aktiven Zustand übergeht, und wobei das Anforderungssignal für die Daten, die den analytbezogenen Signalen zugeordnet sind, die von dem entfernten Ort empfangen werden, ein Radiofrequenz- (RF) Steuersignal umfasst.

10. Baugruppe (110, 211) nach Anspruch 9, wobei die HF-Leistung und das HF-Steuersignal beide auf demselben Trägersignal von dem entfernten Ort (220) empfangen werden.

11. Baugruppe (110, 211) nach Anspruch 10, wobei die Sensorantenne abgestimmt ist, so dass, wenn die Datenverarbeitungskomponente (610, 950) bei Erfassung der HF-Energie von dem entfernten Ort (220) von dem inaktiven Zustand in den aktiven Zustand übergeht, die Daten, die den analytbezogenen Signalen zugeordnet sind, als reflektiertes Rückstreusignal zu dem entfernten Ort übertragen werden.

12. Baugruppe (110, 211) nach Anspruch 1, wobei der Analytsensor (101, 250, 540, 1020) eine gebogene Konfiguration aufweist, wodurch mindestens der proximale Teil des Körpers des Analytsensors in einer Richtung im Wesentlichen planar zur Oberfläche der Haut gehalten wird.

## Revendications

1. Assemblage de dispositif de surveillance d'analyte intégré (110, 211), comprenant :
un détecteur d'analyte (101, 250, 540, 1020) pour le positionnement transcutané à travers une couche de peau (210) et maintenu en contact fluidique avec un fluide interstitiel sous la couche de peau pendant une période de temps prédéterminée, le détecteur d'analyte ayant une partie proximale et une partie distale ; et
des éléments électroniques de détecteur (102, 1030) couplés au détecteur d'analyte, les éléments électroniques de détecteur comprenant :
une carte de circuit (1310) ayant une couche conductrice (801) et une antenne de détecteur disposée sur la couche conductrice ;
un ou plusieurs contacts électriques (1360) fournis sur la carte de circuit et couplés avec la partie proximale du détecteur d'analyte pour maintenir une communication électrique continue ; et
un composant de traitement de données (610, 950) incluant un circuit intégré propre à une application (ASIC) fourni sur la carte de circuit et en communication de signaux avec le détecteur d'analyte, le composant de traitement de données configuré pour exécuter une ou plusieurs routines pour traiter les signaux liés à l'analyte reçus du détecteur d'analyte, le composant de traitement de données configuré pour commander la transmission des données associées aux signaux liés à l'analyte reçus du détecteur d'analyte vers un lieu distant (220) en utilisant l'antenne de détecteur uniquement en réponse à un signal de demande reçu du lieu distant ;
**caractérisé en ce que** le détecteur d'analyte est couplé avec les éléments électroniques du détecteur et fourni à l'utilisateur au sein d'un boîtier (1010) avant le positionnement du détecteur d'analyte à travers la couche de peau.

2. Assemblage (110, 211) selon la revendication 1 dans lequel la partie proximale du détecteur d'analyte (101, 250, 540) et la carte de circuit (1310) sont encapsulées.

3. Assemblage (110, 211) selon la revendication 2 dans lequel la partie proximale du détecteur d'analyte (101, 250, 540, 1020) et la carte de circuit (1310) sont encapsulées avec un matériau d'empotage.

4. Assemblage (110, 211) selon la revendication 1 dans lequel la carte de circuit (1310) inclut une couche supérieure et une couche inférieure, où la couche conductrice (801) est disposée entre la couche supérieure et la couche inférieure.

5. Assemblage (110, 211) selon la revendication 1 dans lequel l'antenne inclut une antenne cadre ou une antenne dipolaire.

6. Assemblage (110, 211) selon la revendication 1 incluant une alimentation en puissance (1120) pour fournir de la puissance aux éléments électroniques de détecteur.

7. Assemblage (110, 211) selon la revendication 1 dans lequel le composant de traitement de données inclut une machine à états.

8. Assemblage (110, 211) selon la revendication 7 dans lequel la machine à états est configurée pour exécuter une ou plusieurs logiques programmées ou programmables pour traiter les données de signaux associées aux signaux liés à l'analyte reçus du détecteur d'analyte (101, 250, 540, 1020).

9. Assemblage (110, 211) selon la revendication 1 dans lequel le composant de traitement de données (610, 950) est configuré pour effectuer la transition d'un état inactif à un état actif à la détection d'une puissance RF provenant du lieu distant (220), et dans lequel le signal de demande pour les données associées aux signaux liés à l'analyte reçus du lieu distant inclut un signal de commande radiofréquence (RF).

10. Assemblage (110, 211) selon la revendication 9 dans lequel la puissance RF et le signal de commande RF sont tous les deux reçus sur le même signal porteur provenant du lieu distant (220).

11. Assemblage (110, 211) selon la revendication 10 dans lequel l'antenne de détecteur est accordée de sorte que lorsque le composant de traitement de données (610, 950) effectue une transition de l'état inactif à l'état actif à la détection de la puissance RF provenant du lieu distant (220), les données associées aux signaux liés à l'analyte sont transmises comme un signal rétrodiffusé réfléchi vers le lieu distant.

12. Assemblage (110, 211) selon la revendication 1 dans lequel le détecteur d'analyte (101, 250, 540, 1020) inclut une configuration courbée, par laquelle au moins la partie proximale du corps du détecteur d'analyte est maintenue dans une direction substantiellement plane par rapport à la surface de la peau.
